(19) Europäisches Patentamt
European Patent Office
Office européen des brevets

(11) EP 4 495 120 A1

(12) # EUROPEAN PATENT APPLICATION
published in accordance with Art. 153(4) EPC

(43) Date of publication:
**22.01.2025 Bulletin 2025/04**

(21) Application number: **23769911.1**

(22) Date of filing: **17.03.2023**

(51) International Patent Classification (IPC):
***C07D 491/107*** *(2006.01)* ***C07C 59/255*** *(2006.01)*
***C07C 51/41*** *(2006.01)* ***A61K 31/444*** *(2006.01)*
***A61P 37/02*** *(2006.01)* ***A61P 37/08*** *(2006.01)*
***A61P 29/00*** *(2006.01)* ***A61P 31/00*** *(2006.01)*
***A61P 3/00*** *(2006.01)* ***A61P 9/00*** *(2006.01)*
***A61P 11/00*** *(2006.01)* ***A61P 13/12*** *(2006.01)*
***A61P 17/00*** *(2006.01)*

(52) Cooperative Patent Classification (CPC):
**A61K 31/444; A61P 1/00; A61P 1/02; A61P 1/04; A61P 1/16; A61P 3/00; A61P 7/00; A61P 9/00; A61P 11/00; A61P 13/12; A61P 15/00; A61P 17/00; A61P 29/00; A61P 31/00; A61P 37/02;** (Cont.)

(86) International application number:
**PCT/CN2023/082191**

(87) International publication number:
**WO 2023/174409 (21.09.2023 Gazette 2023/38)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.03.2022 CN 202210272562**
**09.09.2022 CN 202211104893**

(71) Applicant: **Shanghai Meiyue Biotech Development Co., Ltd.**
**Shanghai 200120 (CN)**

(72) Inventors:
- **YAO, Yuanshan**
  **Shanghai 200120 (CN)**
- **TIAN, Yong**
  **Shanghai 200120 (CN)**
- **LUAN, Linbo**
  **Shanghai 200120 (CN)**
- **CHEN, Yongkai**
  **Shanghai 200120 (CN)**
- **WANG, Chaodong**
  **Shanghai 200120 (CN)**

(74) Representative: **Dai, Simin**
**Reyda IP**
**A073**
**157, Quai du Président Roosevelt**
**92130 Issy-les-Moulineaux (FR)**

(54) **SALT FORM AND CRYSTAL FORM OF VANIN ENZYME INHIBITOR, METHOD FOR PREPARING SAME, AND USE THEREOF**

(57) The present invention relates to a salt form and a crystal form of a Vanin enzyme inhibitor represented by formula I, a method for preparing same, and use thereof. The salt form is a salt formed by the compound represented by formula I with an acid, and the crystal form is selected from a crystal form A, a crystal form B, a crystal form C, and a crystal form D of an L-tartrate of the compound represented by formula I. The salt of the compound represented by formula I of the present invention features high stability and good water solubility, which is conducive to the enhancement of oral absorbability, thus improving the bioavailability; and the preparation method is simple in operation, easy to control, good in reproducibility, and suitable for industrial production. The crystal form of the present invention features high stability, good solubility, and low hygroscopicity, and has promising prospects for being developed into medicaments.

EP 4 495 120 A1

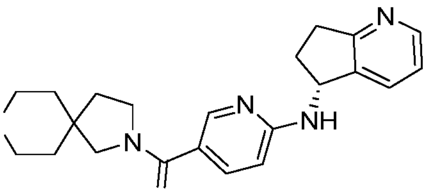

I

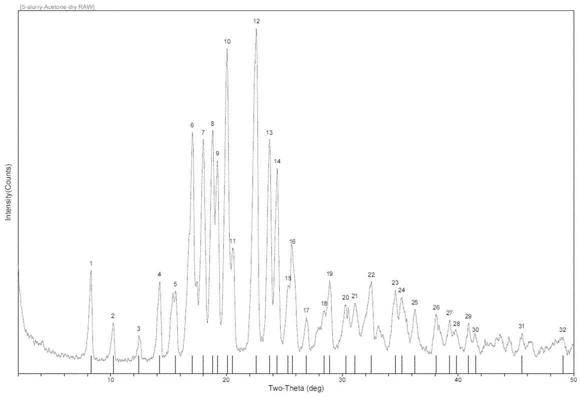


Fig. 1

(52) Cooperative Patent Classification (CPC): (Cont.)
**A61P 37/06; A61P 37/08; C07C 51/41; C07C 59/255; C07D 491/107**

## Description

The present application claims the right of the priorities of Chinese patent application CN202210272562.6 filed on March 18, 2022 and Chinese patent application CN202211104893.5 filed on September 9, 2022. The contents of these applications are incorporated herein by reference in their entireties.

TECHNICAL FIELD

The present disclosure belongs to the field of pharmaceuticals, and specifically relates to a salt form and a crystal form of a Vanin enzyme inhibitor, a preparation method therefor, and a use of the salt form and the crystal form for the prevention and/or treatment of cardiovascular diseases and tumor diseases.

BACKGROUND

Vanin-1 (vascular non-inflammatory molecule-1) is an exonuclease with pantetheinase activity, which mainly catalyzes the hydrolysis of pantetheine to produce pantothenic acid (VB5) and mercaptoethylamine. Coenzyme A (CoA), synthesized from VB5, regulates biotransformations such as fatty acid synthesis and oxidation as well as energy metabolism, while the reversible reaction between mercaptoethylamine and cystamine is an important sensor of oxidative stress. A growing number of studies have found that deficiency or reduced levels of mercaptoethylamine lead to enhanced γ-GCS activity, causing elevated endogenous GSH reserves in tissues, which may thereby prevent or eliminate tissue inflammation. Studies have found that mRNA of Vanin-1 is highly expressed in human colon, duodenum, endometrium, liver, kidney, gallbladder, and small intestine. In patients with UC (ulcerative colitis), Vanin-1 was highly expressed and diffuse, and limited to brush borders. In addition, the expression level of Vanin-1 in the colon was still significantly higher than that of the control group during the clinically quiescent phase of UC. In the TNBS model experiment, the survival rate of Vanin-1 knockout (Vanin-$1^{-/-}$) mice was significantly higher than that of the model control group, and there was no significant weight loss. Moreover, 90% of Vanin-$1^{-/-}$ mice treated with cystamine died within 5 days, indicating that cystamine completely reversed the protective effect of Vanin-1 deficiency on colitis. In addition, histopathological analysis of mice has found that the inhibition or knockout of Vanin-1 can significantly ameliorate the colon lesions in mice (Berruyer C, et al., Vanin-1-/- mice exhibit a glutathione mediated tissue resistance to oxidative stress. Mol. Cell Biol. 2004; 24: 7214-7224; Berruyer C, et al., Vanin-1 licenses inflammatory mediator production by gut epithelial cells and controls colitis by antagonizing peroxisome proliferator-activated receptor γ activity. J. Exp. Med. 2006; 203: 2817-2827).

Furthermore, Vanin-1 is also considered to play a regulatory role in cardiovascular diseases and tumor diseases. Studies have demonstrated that Vanin-1 regulates the activation of smooth muscle cells *in vitro* and the development of neointimal hyperplasia in response to carotid artery ligation *in vivo.* VNN1 gene polymorphisms are associated with blood pressure and HDL levels. In SF-1 transgenic mice, Vanin-1 deficiency prevented the mice from developing neoplasia of the adrenal cortex, suggesting a role for Vanin-1 in certain cancers. Studies in inflammatory diseases have found that Vanin-1 is highly upregulated in psoriatic skin lesions compared to normal individuals. Gene expression of VNN1 was also upregulated in whole blood from patients with childhood immune thrombocytopenia (ITP), wherein overexpression of VNN1 was associated with the progression of chronic ITP. In addition, elevated Vanin-1 has been detected in the urine of patients with a variety of renal disorders, including systemic lupus erythematosus, nephrotoxicant-induced renal injury, and type 2 diabetes (Rommelaere S, et al., PPARalpha regulates the production of serum Vanin-1 by liver. FEBS Lett. 2013 Nov 15; 587(22): 3742-8).

Chinese patent application CN2021110954656 (WO2022063197A1) discloses a structure of formula I as follows:

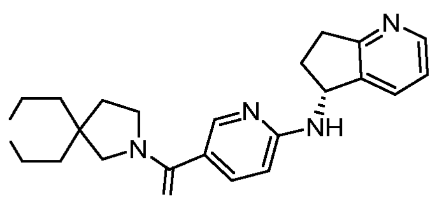

I

The compound of formula I is an effective Vanin enzyme inhibitor and has broad medicinal prospects. Thus, there is a need for research and development on an efficient, low-toxic and/or long-acting pharmaceutically acceptable active ingredient to ameliorate the above technical problems.

SUMMARY

In order to solve the problems in the prior art, a first aspect of the present disclosure provides a pharmaceutically acceptable salt of a compound of formula I. The compound of formula I is as follows:

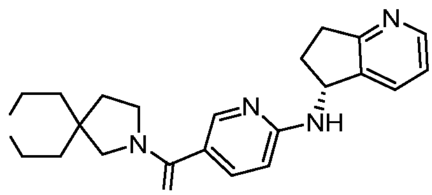

I

The pharmaceutically acceptable salt of the compound of formula I is a salt formed by the compound of formula I with an acid.

The acid is selected from an inorganic acid or an organic acid, such as hydrochloric acid, hydrofluoric acid, hydrobromic acid, hydroiodic acid, sulfuric acid, pyrosulfuric acid, phosphoric acid, nitric acid, formic acid, acetic acid, acetoacetic acid, pyruvic acid, trifluoroacetic acid, propionic acid, butyric acid, hexanoic acid, heptanoic acid, undecanoic acid, lauric acid, benzoic acid, salicylic acid, 2-(4-hydroxybenzoyl)benzoic acid, camphoric acid, cinnamic acid, cyclopentylpropionic acid, digluconic acid, 3-hydroxy-2-naphthoic acid, nicotinic acid, pamoic acid, pectic acid, persulfuric acid, 3-phenylpropionic acid, picric acid, pivalic acid, 2-hydroxyethanesulfonic acid, itaconic acid, sulfamic acid, trifluoromethanesulfonic acid, dodecyl sulfuric acid, ethanesulfonic acid, benzenesulfonic acid, p-toluenesulfonic acid, methanesulfonic acid, 2-naphthalenesulfonic acid, naphthalenedisulfonic acid, camphorsulfonic acid, citric acid, L-tartaric acid, stearic acid, lactic acid, oxalic acid, malonic acid, succinic acid, malic acid, adipic acid, alginic acid, maleic acid, fumaric acid, *D*-gluconic acid, mandelic acid, ascorbic acid, glucoheptanoic acid, glycerophosphoric acid, aspartic acid, sulfosalicylic acid, hemi-sulfuric acid, or thiocyanic acid. As an example, the acid may be selected from one of hydrochloric acid, hydrobromic acid, sulfuric acid, phosphoric acid, nitric acid, methanesulfonic acid, *p*-toluenesulfonic acid, fumaric acid, maleic acid, citric acid, *L*-tartaric acid, succinic acid, ethanesulfonic acid, *L*-malic acid, *L*-glutamic acid, oxalic acid, *D*-malic acid, pamoic acid, oxalic acid, formic acid, acetic acid, trifluoroacetic acid, lauric acid, benzoic acid, and benzenesulfonic acid.

In a preferred embodiment, the pharmaceutically acceptable salt of the compound of formula I is selected from one of hydrochloride, sulfate, phosphate, methanesulfonate, *p*-toluenesulfonate, fumarate, maleate, citrate, *L*-tartrate, succinate, ethanesulfonate, *L*-malate, *L*-glutamate, oxalate, *D*-malate, and pamoate thereof.

In a preferred embodiment, in the pharmaceutically acceptable salt of a compound of formula I, the molar ratio of the compound of formula I to the acid may be selected from 1:1, 2:1, or 3:1, provided that ions of the compound of formula I in the salt are charge balanced with ions of the acid. For example, when the number of ionizable hydrogen atoms in the acid (*e.g.,* hydrochloric acid, methanesulfonic acid, *p*-toluenesulfonic acid, ethanesulfonic acid) is 1, the molar ratio of the compound of formula I to the acid is 1: 1; when the number of ionizable hydrogen atoms in the acid (*e.g.,* sulfuric acid, fumaric acid, maleic acid, citric acid, *L*-tartaric acid, oxalic acid, succinic acid, malic acid, *L*-glutamic acid, pamoic acid) is 2, the molar ratio of the compound of formula I to the acid may be 1: 1 or 2:1; when the number of ionizable hydrogen atoms in the acid (*e.g.,* phosphoric acid) is 3, the molar ratio of the compound of formula I to the acid is 1:1, 2:1, or 3:1.

In a more preferred embodiment, in the pharmaceutically acceptable salt of the compound of formula I, the molar ratio of the compound of formula I to the acid is 1: 1; that is, when the acid is *L*-tartaric acid, fumaric acid, or malic acid, the pharmaceutically acceptable salt is selected from a monotartrate, a monofumarate, and a monomalate of the compound of formula I, more preferably a mono-*L*-tartrate of the compound of formula I.

A second aspect of the present disclosure provides a preparation method for the pharmaceutically acceptable salt of the compound of formula I, comprising reacting the compound of formula I with the acid to prepare the pharmaceutically acceptable salt of the compound of formula I.

According to an embodiment of the present disclosure, the preparation method comprises dissolving the compound of formula I in an organic solvent A, adding an acid for a reaction, and then adding an organic solvent B to prepare the pharmaceutically acceptable salt of the compound of formula I.

According to an embodiment of the present disclosure, the acid has the definition described above. In some embodiments, the acid is first dissolved in an organic solvent C to prepare an acid solution form before being added to the reaction.

According to an embodiment of the present disclosure, the organic solvent A is selected from at least one of an ester, a ketone, and an alcohol. The ester may be selected from an organic carboxylic acid ester, such as methyl formate, ethyl acetate, isobutyl formate, ethyl propyl acetate, isopropyl acetate, or a combination thereof; the ketone may be

selected from a ketone having 3 to 10 carbon atoms, such as acetone, butanone, pentanone, methyl ethyl ketone, 4-methyl-2-pentanone, or a combination thereof; the alcohol may be selected from an alcohol having 1 to 8 carbon atoms, such as methanol, ethanol, n-propanol, isopropanol, n-butanol, neopentyl alcohol, or a combination thereof.

According to an embodiment of the present disclosure, the organic solvent B is selected from a nitrile, an ester, an ether, or a combination thereof. The nitrile may be selected from a nitrile having 2 to 6 carbon atoms, such as acetonitrile, propionitrile, isopropionitrile, butyronitrile, or a combination thereof; the ester may be selected from an organic carboxylic acid ester, such as methyl formate, ethyl acetate, isobutyl formate, ethyl propyl acetate, isopropyl acetate, or a combination thereof; the ether may be selected from an ether having 2 to 6 carbon atoms, such as diethyl ether, propyl ether, isopropyl ether, tert-butyl ether, methyl tert-butyl ether, or a combination thereof.

According to an embodiment of the present disclosure, it should be understood by those skilled in the art that the organic solvent C is selected from any organic solvent in which the acid is dissolved.

In some embodiments, when the acid is selected from *L*-tartaric acid, the organic solvent C is selected from an alcohol, and the alcohol may be selected from an alcohol having 1 to 8 carbon atoms, such as methanol, ethanol, *n*-propanol, isopropanol, *n*-butanol, neopentyl alcohol, or a combination thereof.

In the preparation method, the organic solvent A and the organic solvent B have a volume ratio of 1:1 to 5, preferably 1:1.

In the preparation method, the compound of formula I and the acid have a molar ratio of 1:0.8 to 1:1.5, preferably 1:0.9 to 1:1.3, more preferably 1: 1.0 to 1:1.1.

According to an embodiment of the present disclosure, the reaction is at a temperature is 20°C to 80°C, preferably 20°C to 60°C.

According to an embodiment of the present disclosure, the preparation method further comprises the steps of filtering and/or drying after the reaction is completed, so as to prepare the pharmaceutically acceptable salt of the compound of formula I.

In the preparation method, the drying may be at a temperature of 30°C to 60°C, more preferably 40°C to 50°C.

In the preparation method, the drying is at a pressure of 0 to 20 KPa, preferably 0 to 10 KPa, more preferably 5 to 10 KPa.

The present disclosure also provides a crystal, preferably a single crystal of the mono-L-tartrate of the compound of formula I. Unit cell parameters of the single crystal are as follows:

Monoclinic crystal system with space group $P2_1$,

$$a = 6.3430\ (5)\ \text{Å},$$

$$b = 8.8372\ (7)\ \text{Å},\ \beta = 96.577,$$

$$c = 24.809\ (2)\ \text{Å},$$

$$V = 1381.5\ (2)\ \text{Å}^3,\ Z = 2.$$

The present disclosure also provides a preparation method for the crystal, especially the single crystal of the mono-*L*-tartrate of the compound of formula I, comprising dissolving the mono-*L*-tartrate of the compound of formula I in a solvent D and then diffusing in an atmosphere of a solvent E.

The solvent D is selected from an alcohol solvent, such as methanol, ethanol, *n*-propanol, isopropanol, *n*-butanol, neopentyl alcohol, or a combination thereof.

The solvent E is selected from an ester solvent, an ether solvent, an alkane solvent, or a combination thereof. The ester solvent may be selected from an organic carboxylic acid ester, such as ethyl acetate and isopropyl acetate; the ether may be selected from an ether having 2 to 6 carbon atoms, such as diethyl ether, propyl ether, isopropyl ether, *tert*-butyl ether, and methyl tert-butyl ether; the alkane may be selected from a hydrocarbon having 1 to 8 carbon atoms, such as *n*-hexane and *n*-heptane.

A third aspect of the present disclosure provides a crystal form of the pharmaceutically acceptable salt of the compound of formula I.

According to a preferred embodiment of the present disclosure, provided is a crystal form of the mono-*L*-tartrate of the compound of formula I, selected from a crystal form A, a crystal form B, a crystal form C, and a crystal form D as described below.

In some embodiments, provided is a crystal form A of the mono-*L*-tartrate of the compound of formula I. The

crystal form A has characteristic peaks at 17.06 ± 0.20°, 20.06 ± 0.20°, 22.58 ± 0.20° in an X-ray powder diffraction expressed at a 2θ angle using Cu-Kα radiation.

Preferably, the crystal form A has characteristic peaks at 17.06 ± 0.20°, 18.00 ± 0.20°, 18.80 ± 0.20°, 19.22 ± 0.20°, 20.06 ± 0.20°, 22.58 ± 0.20°, 23.72 ± 0.20°, 24.38 ± 0.20° in an X-ray powder diffraction expressed at a 2θ angle using Cu-Kα radiation.

Preferably, the crystal form A has characteristic peaks at 8.30 ± 0.20°, 14.24 ± 0.20°, 17.06 ± 0.20°, 18.00 ± 0.20°, 18.80 ± 0.20°, 19.22 ± 0.20°, 20.06 ± 0.20°, 20.52 ± 0.20°, 22.58 ± 0.20°, 23.72 ± 0.20°, 24.38 ± 0.20°, 25.70 ± 0.20° in an X-ray powder diffraction expressed at a 2θ angle using Cu-Kα radiation.

Preferably, the crystal form A has an X-ray powder diffraction expressed at a 2θ angle using Cu-Kα radiation as shown in Table 1, with an error range of ± 0.20°:

Table 1: XRPD analysis data of crystal form A

| Peak number | 2θ [°] | Relative intensity% | Peak number | 2θ [°] | Relative intensity% |
|---|---|---|---|---|---|
| 1 | 8.30 | 28.3 | 17 | 26.90 | 10.2 |
| 2 | 10.22 | 11.9 | 18 | 28.44 | 11.9 |
| 3 | 12.44 | 8 | 19 | 28.92 | 19.4 |
| 4 | 14.24 | 24.2 | 20 | 30.30 | 9.1 |
| 5 | 15.60 | 20.4 | 21 | 31.10 | 8.4 |
| 6 | 17.06 | 70.8 | 22 | 32.52 | 17.2 |
| 7 | 18.00 | 54.4 | 23 | 34.60 | 15.7 |
| 8 | 18.80 | 60 | 24 | 35.16 | 13.4 |
| 9 | 19.22 | 51 | 25 | 36.28 | 10.7 |
| 10 | 20.06 | 95.8 | 26 | 38.12 | 11 |
| 11 | 20.52 | 32.6 | 27 | 39.28 | 8.2 |
| 12 | 22.58 | 100 | 28 | 39.88 | 5.6 |
| 13 | 23.72 | 63.4 | 29 | 40.90 | 8.7 |
| 14 | 24.38 | 55.2 | 30 | 41.54 | 4.8 |
| 15 | 25.32 | 18.7 | 31 | 45.52 | 6.2 |
| 16 | 25.70 | 31.1 | 32 | 49.06 | 4.4 |

Preferably, the crystal form A has an X-ray powder diffraction pattern substantially as shown in Fig. 1.

According to an embodiment of the present disclosure, the crystal form A is an anhydride of the mono-L-tartrate of the compound of formula I.

According to an embodiment of the present disclosure, the crystal form A has a first endothermic peak near a peak temperature of 150.14°C when heated, as shown by differential scanning calorimetry (DSC) analysis.

Preferably, the crystal form A has a DSC pattern substantially as shown in Fig. 2.

According to an embodiment of the present disclosure, the crystal form A has a weight loss of approximately 0.069% in a range of 22.03°C to 120°C, as shown by thermogravimetric analysis (TGA).

Preferably, the crystal form A has a TGA pattern substantially as shown in Fig. 3.

According to an embodiment of the present disclosure, the crystal form A is a crystal with irregular morphology. Preferably, the crystal form A has a particle size of 10 μm or less.

Preferably, the crystal form A has a PLM pattern substantially as shown in Fig. 4.

In some embodiments, provided is a crystal form B of the mono-L-tartrate of the compound of formula I. The crystal form B has characteristic peaks at 19.28 ± 0.20°, 19.94 ± 0.20°, 21.30 ± 0.20°, 23.72 ± 0.20° in an X-ray powder diffraction expressed at a 2θ angle using Cu-Kα radiation.

Preferably, the crystal form B has characteristic peaks at 7.56 ± 0.20°, 17.36 ± 0.20°, 19.28 ± 0.20°, 19.94 ± 0.20°, 21.30 ± 0.20°, 23.72 ± 0.20°, 26.02 ± 0.20° in an X-ray powder diffraction expressed at a 2θ angle using Cu-Kα radiation.

Preferably, the crystal form B has characteristic peaks at 7.56 ± 0.20°, 17.36 ± 0.20°, 18.14 ± 0.20°, 19.28 ± 0.20°, 19.94 ± 0.20°, 21.30 ± 0.20°, 23.72 ± 0.20°, 24.52 ± 0.20°, 26.02 ± 0.20°, 29.52 ± 0.20° in an X-ray powder

diffraction expressed at a 2θ angle using Cu-Kα radiation.

Preferably, the crystal form B has characteristic peaks at 3.78 ± 0.20°, 7.56 ± 0.20°, 17.36 ± 0.20°, 18.14 ± 0.20°, 19.28 ± 0.20°, 19.94 ± 0.20°, 21.30 ± 0.20°, 23.72 ± 0.20°, 24.52 ± 0.20°, 26.02 ± 0.20°, 29.52 ± 0.20° in an X-ray powder diffraction expressed at a 2θ angle using Cu-Kα radiation.

Preferably, the crystal form B has an X-ray powder diffraction expressed at a 2θ angle using Cu-Kα radiation as shown in Table 2, with an error range of ± 0.20°:

Table 2: XRPD analysis data of crystal form B

| Peak number | 2θ [°] | Relative intensity% | Peak number | 2θ [°] | Relative intensity% |
|---|---|---|---|---|---|
| 1 | 3.78 | 23 | 21 | 26.72 | 22 |
| 2 | 7.56 | 58.2 | 22 | 28.56 | 15.6 |
| 3 | 10.62 | 7.7 | 23 | 29.52 | 37.9 |
| 4 | 14.16 | 16.5 | 24 | 30.66 | 16.5 |
| 5 | 15.06 | 19.6 | 25 | 31.20 | 15.4 |
| 6 | 15.62 | 14.1 | 26 | 33.16 | 17.5 |
| 7 | 16.48 | 22.4 | 27 | 33.78 | 17 |
| 8 | 17.36 | 60.1 | 28 | 34.58 | 12.7 |
| 9 | 18.14 | 27.2 | 29 | 35.12 | 20.4 |
| 10 | 18.58 | 21.6 | 30 | 35.60 | 8.6 |
| 11 | 19.28 | 100 | 31 | 36.42 | 7.9 |
| 12 | 19.94 | 98.2 | 32 | 38.20 | 11.3 |
| 13 | 21.30 | 72.9 | 33 | 39.60 | 4 |
| 14 | 22.56 | 16 | 34 | 40.36 | 7.3 |
| 15 | 22.90 | 4.6 | 35 | 41.02 | 3.5 |
| 16 | 23.72 | 70.9 | 36 | 42.20 | 5.8 |
| 17 | 24.52 | 28 | 37 | 43.98 | 8.5 |
| 18 | 25.00 | 14 | 38 | 45.08 | 6.7 |
| 19 | 25.42 | 11.3 | 39 | 47.32 | 6.1 |
| 20 | 26.02 | 51.3 | | | |

Preferably, the crystal form B has an X-ray powder diffraction pattern substantially as shown in Fig. 5.

According to an embodiment of the present disclosure, the crystal form B is a hydrate of the mono-*L*-tartrate of the compound of formula I.

According to an embodiment of the present disclosure, the hydrate contains 0.5 to 1 mol of water.

According to an embodiment of the present disclosure, the crystal form B has a first endothermic peak near a peak temperature of 61.57°C when heated and a second endothermic peak near a peak temperature of 152.18°C when heated, as shown by differential scanning calorimetry (DSC) analysis. The first endothermic peak is a dehydration peak, and the second endothermic peak is a melting peak.

Preferably, the crystal form B has a DSC pattern substantially as shown in Fig. 6.

According to an embodiment of the present disclosure, the crystal form B has a weight loss of approximately 2.76% in a range of 21.34°C to 120°C, as shown by thermogravimetric analysis (TGA).

Preferably, the crystal form B has a TGA pattern substantially as shown in Fig. 7.

In some embodiments, provided is a crystal form C of the mono-L-tartrate of the compound of formula I. The crystal form C has characteristic peaks at 17.88 ± 0.20°, 19.40 ± 0.20°, 21.38 ± 0.20° in an X-ray powder diffraction expressed at a 2θ angle using Cu-Kα radiation.

Preferably, the crystal form C has characteristic peaks at 7.14 ± 0.20°, 17.88 ± 0.20°, 19.40 ± 0.20°, 20.06 ± 0.20°, 21.38 ± 0.20°, 23.76 ± 0.20°, 25.92 ± 0.20° in an X-ray powder diffraction expressed at a 2θ angle using Cu-Kα radiation.

Preferably, the crystal form C has characteristic peaks at 3.58 ± 0.20°, 7.14 ± 0.20°, 13.96 ± 0.20°, 17.10 ±

0.20°, 17.88 ± 0.20°, 19.40 ± 0.20°, 20.06 ± 0.20°, 21.38 ± 0.20°, 25.92 ± 0.20°, 29.38 ± 0.20° in an X-ray powder diffraction expressed at a 2θ angle using Cu-Kα radiation.

Preferably, the crystal form C has an X-ray powder diffraction expressed at a 2θ angle using Cu-Kα radiation as shown in Table 3, with an error range of ± 0.20°:

Table 3: XRPD analysis data of crystal form C

| Peak number | 2θ [°] | Relative intensity% | Peak number | 2θ [°] | Relative intensity% |
|---|---|---|---|---|---|
| 1 | 3.58 | 28.6 | 13 | 24.38 | 12.5 |
| 2 | 7.14 | 51.6 | 14 | 25.00 | 7.7 |
| 3 | 10.46 | 17.3 | 15 | 25.92 | 36.6 |
| 4 | 12.22 | 9.2 | 16 | 27.58 | 9.1 |
| 5 | 13.96 | 26.5 | 17 | 28.06 | 11 |
| 6 | 14.56 | 17.5 | 18 | 29.38 | 21 |
| 7 | 17.10 | 35 | 19 | 30.22 | 5.1 |
| 8 | 17.88 | 77.1 | 20 | 30.78 | 12.3 |
| 9 | 19.40 | 75 | 21 | 33.66 | 9.1 |
| 10 | 20.06 | 48.8 | 22 | 35.24 | 8.9 |
| 11 | 21.38 | 100 | 23 | 37.40 | 5.6 |
| 12 | 23.76 | 49.1 | 24 | 38.86 | 4.2 |

Preferably, the crystal form C has an X-ray powder diffraction pattern substantially as shown in Fig. 8.

According to an embodiment of the present disclosure, the crystal form C is a solvate of the mono-*L*-tartrate of the compound of formula I, preferably an ethanol solvate of the mono-*L*-tartrate of the compound of formula I.

According to an embodiment of the present disclosure, the crystal form C has a first endothermic peak near a peak temperature of 129.45°C when heated and a second endothermic peak near a peak temperature of 151.90°C when heated, as shown by differential scanning calorimetry (DSC) analysis. The first endothermic peak is a desolvation peak, and the second endothermic peak is a melting peak.

Preferably, the crystal form C has a DSC pattern substantially as shown in Fig. 9.

According to an embodiment of the present disclosure, the crystal form C has a weight loss of approximately 4.59% in a range of 21.47°C to 150°C, as shown by thermogravimetric analysis (TGA).

Preferably, the crystal form C has a TGA pattern substantially as shown in Fig. 10.

According to an embodiment of the present disclosure, the crystal form C is a crystal with irregular morphology. Preferably, the crystal form C has a particle size of 10 μm or less.

Preferably, the crystal form C has a PLM pattern substantially as shown in Fig. 11.

In some embodiments, provided is a crystal form D of the mono-*L*-tartrate of the compound of formula I. The crystal form D has characteristic peaks at 3.50 ± 0.20°, 7.46 ± 0.20°, 23.04 ± 0.20° in an X-ray powder diffraction expressed at a 2θ angle using Cu-Kα radiation.

Preferably, the crystal form D has characteristic peaks at 3.50 ± 0.20°, 6.92 ± 0.20°, 7.46 ± 0.20°, 17.22 ± 0.20°, 18.20 ± 0.20°, 19.88 ± 0.20°, 23.04 ± 0.20° in an X-ray powder diffraction expressed at a 2θ angle using Cu-Kα radiation.

Preferably, the crystal form D has characteristic peaks at 3.50 ± 0.20°, 6.92 ± 0.20°, 7.46 ± 0.20°, 17.22 ± 0.20°, 18.20 ± 0.20°, 19.88 ± 0.20°, 20.76 ± 0.20°, 23.04 ± 0.20°, 25.62 ± 0.20° in an X-ray powder diffraction expressed at a 2θ angle using Cu-Kα radiation.

Preferably, the crystal form D has an X-ray powder diffraction expressed at a 2θ angle using Cu-Kα radiation as shown in Table 4, with an error range of ± 0.20°:

Table 4: XRPD analysis data of crystal form D

| Peak number | 2θ [°] | Relative intensity% | Peak number | 2θ [°] | Relative intensity% |
|---|---|---|---|---|---|
| 1 | 3.50 | 87.2 | 10 | 21.88 | 17.8 |
| 2 | 6.92 | 71.3 | 11 | 23.04 | 100 |
| 3 | 7.46 | 78.1 | 12 | 24.50 | 18.8 |

(continued)

| Peak number | 2θ [°] | Relative intensity% | Peak number | 2θ [°] | Relative intensity% |
|---|---|---|---|---|---|
| 4 | 10.38 | 15.7 | 13 | 25.62 | 37.3 |
| 5 | 15.06 | 17.5 | 14 | 26.50 | 14.1 |
| 6 | 17.22 | 71.8 | 15 | 28.22 | 12 |
| 7 | 18.20 | 55.6 | 16 | 28.86 | 12 |
| 8 | 19.88 | 53.8 | 17 | 30.28 | 19.6 |
| 9 | 20.76 | 31.9 | 18 | 32.08 | 19.1 |

Preferably, the crystal form D has an X-ray powder diffraction pattern substantially as shown in Fig. 12.

According to an embodiment of the present disclosure, the crystal form D is a solvate of the mono-*L*-tartrate of the compound of formula I, preferably a tetrahydrofuran solvate of the mono-L-tartrate of the compound of formula I.

According to an embodiment of the present disclosure, the crystal form D has a first endothermic peak near a peak temperature of 94.15°C when heated, a second endothermic peak near a peak temperature of 118.79°C when heated, and a third endothermic peak near a peak temperature of 146.04°C when heated, as shown by differential scanning calorimetry (DSC) analysis. The first and second endothermic peaks are desolvation peaks, and the third endothermic peak is a melting peak.

Preferably, the crystal form D has a DSC pattern substantially as shown in Fig. 13.

According to an embodiment of the present disclosure, the crystal form D has a weight loss of approximately 5.25% in a range of 23.19°C to 120°C, as shown by thermogravimetric analysis (TGA).

Preferably, the crystal form D has a TGA pattern substantially as shown in Fig. 14.

A fourth aspect of the present disclosure provides a preparation method for the crystal form of the pharmaceutically acceptable salt of the compound of formula I.

According to a preferred embodiment of the present disclosure, provided is a preparation method for the crystal form of the mono-*L*-tartrate of the compound of formula I.

In some embodiments, provided is a preparation method I for the crystal form A of the mono-*L*-tartrate of the compound of formula I, comprising stirring the mono-*L*-tartrate of the compound of formula I in a solvent to obtain the crystal form A.

The stirring is at a temperature of 20 to 80°C, preferably 25 to 55°C.

The solvent is selected from an alcohol solvent, an ester solvent, a ketone solvent, an ether solvent, an alkane solvent, a halogenated hydrocarbon solvent, and a nitrile solvent, or a combination thereof.

The alcohol solvent is selected from one of methanol, ethanol, and isopropanol.

The ester solvent is selected from one of ethyl acetate, propyl acetate, and isopropyl acetate.

The ketone solvent is selected from one of acetone, 2-butanone, methyl isobutyl ketone, and 4-methyl-2-pentanone.

The ether solvent is selected from one of diethyl ether, propyl ether, isopropyl ether, methyl *tert*-butyl ether, and tetrahydrofuran.

The alkane solvent is selected from one of toluene, *n*-heptane, and cyclohexane.

The nitrile solvent is selected from one of acetonitrile, phenylacetonitrile, and benzonitrile.

The mass/volume ratio of the mono-*L*-tartrate of the compound of formula I to the solvent is 1 g:(20 to 40) mL, preferably 1 g:(20 to 30) mL.

In some embodiments, provided is a preparation method II for the crystal form A of the mono-*L*-tartrate of the compound of formula I, comprising dissolving the mono-L-tartrate of the compound of formula I in an alcohol solvent, followed by stirring with the addition of an anti solvent to obtain the crystal form A.

The alcohol solvent is selected from one of methanol, ethanol, propanol, and isopropanol.

The anti solvent is one or more of an ether solvent or an ester solvent. The ether solvent is selected from one of diethyl ether, propyl ether, isopropyl ether, and methyl *tert*-butyl ether. The ester solvent is selected from one of ethyl acetate, propyl acetate, and isopropyl acetate.

The mass/volume ratio of the mono-L-tartrate of the compound of formula I to the alcohol solvent and the antisolvent is 1 g:(10 to 30) mL:(80 to 120) mL, preferably 1 g:(15 to 25) mL:(90 to 110) mL.

According to an embodiment of the present disclosure, the preparation method I or II for the crystal form A further comprises post-processing steps such as filtration and drying.

In some embodiments, provided is a preparation method I for the crystal form B of the mono-L-tartrate of the compound of formula I, comprising placing the crystal form A under a high humidity condition to obtain the crystal form B.

Preferably, the crystal form A is placed for 2 days or more, more preferably for 3 days or more.

The high humidity condition is 80% to 100% RH, preferably 90% to 100% RH.

In some embodiments, provided is a preparation method II for the crystal form B of the mono-*L*-tartrate of the compound of formula I, comprising dissolving the mono-*L*-tartrate of the compound of formula I in an alcohol solvent and evaporating the solvent to obtain the crystal form B.

The alcohol solvent is selected from one of methanol, ethanol, and isopropanol.

The dissolving is at a temperature of 15 to 45°C, preferably 25 to 35°C.

The mass/volume ratio of the mono-*L*-tartrate of the compound of formula I to the alcohol solvent is 1 g:(10 to 30) mL, preferably 1 g:(15 to 25) mL.

In some embodiments, provided is a preparation method for the crystal form C of the mono-*L*-tartrate of the compound of formula I, comprising dissolving the mono-L-tartrate of the compound of formula I in an alcohol solvent and crystallizing to obtain the crystal form C.

The alcohol solvent is selected from methanol, ethanol, and/or isopropanol, preferably methanol.

The mass/volume ratio of the mono-*L*-tartrate of the compound of formula I to the alcohol solvent is 1 g:(20 to 40) mL, preferably 1 g:(20 to 30) mL.

According to an embodiment of the present disclosure, the preparation method for the crystal form C further comprises post-processing steps such as filtration and drying.

In some embodiments, provided is a preparation method for the crystal form D of the mono-L-tartrate of the compound of formula I, comprising dissolving the mono-*L*-tartrate of the compound of formula I in an ether solvent, heating with stirring, and cooling to precipitate a solid to obtain the crystal form D.

The ether solvent is selected from one of diethyl ether, tetrahydrofuran, and methyl tert-butyl ether, preferably tetrahydrofuran.

The heating is at a temperature of 30°C to 80°C, preferably 45 to 75°C.

The cooling is at a temperature of 20°C to 30°C.

The mass/volume ratio of the mono-*L*-tartrate of the compound of formula I to the ether solvent is 1 g:(20 to 40) mL, preferably 1 g:(20 to 30) mL.

According to an embodiment of the present disclosure, the preparation method for the crystal form D comprises post-processing steps such as filtration and drying.

The present disclosure also provides a pharmaceutical composition comprising the pharmaceutically acceptable salt of the compound of formula I or the crystal form thereof, and optionally a pharmaceutically acceptable pharmaceutical excipient. According to a preferred embodiment of the present disclosure, the pharmaceutically acceptable salt is the mono-L-tartrate of the compound of formula I; the crystal form is selected from the crystal form A, crystal form B, crystal form C, and crystal form D of the mono-L-tartrate of the compound of formula I. Preferably, the pharmaceutical composition is in the form of a preparation.

The present disclosure also provides a preparation comprising the pharmaceutically acceptable salt of the compound of formula I or the crystal form thereof, and optionally the pharmaceutically acceptable pharmaceutical excipient. According to a preferred embodiment of the present disclosure, the pharmaceutically acceptable salt is the mono-*L*-tartrate of the compound of formula I; the crystal form is selected from the crystal form A, crystal form B, crystal form C, and crystal form D of the mono-*L*-tartrate of the compound of formula I.

The present disclosure also provides a use of the pharmaceutically acceptable salt of the compound of formula I or the crystal form thereof, or the pharmaceutical composition in the manufacture of a drug for preventing and/or treating a disease or condition related to a Vanin enzyme inhibitor.

According to an embodiment of the present disclosure, the disease or condition related to the Vanin enzyme inhibitor includes one or more of autoimmune disease, inflammatory disease, allergic disease, metabolic disease, infection-based disease, fibrotic disease, cardiovascular disease, respiratory disease, renal disease, dermatological disease, hepatic disease, gastrointestinal disease, oral disease, and hematopoietic disease; for another example, Crohn's disease, inflammatory bowel disease, and ulcerative colitis.

The present disclosure also provides a preventive and/or therapeutic method for the disease or condition related to the Vanin enzyme inhibitor, comprising administering to an individual in need thereof a therapeutically effective amount of the pharmaceutically acceptable salt of the compound of formula I or the crystal form thereof, or the pharmaceutical composition.

According to an embodiment of the present disclosure, the disease or condition related to the Vanin enzyme inhibitor includes one or more of autoimmune disease, inflammatory disease, allergic disease, metabolic disease, infection-based disease, fibrotic disease, cardiovascular disease, respiratory disease, renal disease, dermatological disease, hepatic disease, gastrointestinal disease, oral disease, and hematopoietic disease; for another example, Crohn's disease, inflammatory bowel disease, and ulcerative colitis.

The therapeutic method of the present disclosure may comprise administering one, two, or more of the pharmaceutically acceptable salts of the compound of formula I of the present disclosure or the crystal forms thereof

alone, and administering one, two, or more of the pharmaceutically acceptable salts of the compound of formula I of the present disclosure or the crystal forms thereof in combination with other chemotherapeutic agents. Combination administration allows different drugs to be administered simultaneously or sequentially.

It can be understood by those skilled in the art that the terms "... or a combination thereof" and "one or more of ..." used herein are equivalent to "one, two, or more of ...", and indicate that each of the options and each combination of two or more of the options may be used (*i.e.,* a combination of two of the options is not excluded).

**Beneficial effects**

(1) The salt of the compound of formula I of the present disclosure, especially the mono-L-tartrate, has high stability and high solubility in water, which is conducive to the enhancement of oral absorbability, thus improving the bioavailability;
(2)The preparation method of the salt of the compound of formula I of the present disclosure is simple in operation, easy to control, good in reproducibility, and suitable for industrial production;
(3) The four crystal forms of the mono-*L*-tartrate of the compound of formula I of the present disclosure have high stability, good solubility, and low hygroscopicity, and have promising prospects for drug formation;
(4) The preparation method of the four crystal forms of the mono-*L*-tartrate of the compound of formula I of the present disclosure is simple, featuring mild reaction conditions and high yield of product, which is conducive to industrial production.

BRIEF DESCRIPTION OF THE DRAWINGS

Fig. 1 is the XRPD pattern of the crystal form A of the mono-*L*-tartrate of the compound of formula I.
Fig. 2 is the DSC pattern of the crystal form A of the mono-*L*-tartrate of the compound of formula I.
Fig. 3 is the TGA pattern of the crystal form A of the mono-*L*-tartrate of the compound of formula I.
Fig. 4 is the PLM pattern of the crystal form A of the mono-*L*-tartrate of the compound of formula I.
Fig. 5 is the XRPD pattern of the crystal form B of the mono-*L*-tartrate of the compound of formula I.
Fig. 6 is the DSC pattern of the crystal form B of the mono-*L*-tartrate of the compound of formula I.
Fig. 7 is the TGA pattern of the crystal form B of the mono-L-tartrate of the compound of formula I.
Fig. 8 is the XRPD pattern of the crystal form C of the mono-L-tartrate of the compound of formula I.
Fig. 9 is the DSC pattern of the crystal form C of the mono-L-tartrate of the compound of formula I.
Fig. 10 is the TGA pattern of the crystal form C of the mono-L-tartrate of the compound of formula I.
Fig. 11 is the PLM pattern of the crystal form C of the mono-L-tartrate of the compound of formula I.
Fig. 12 is the XRPD pattern of the crystal form D of the mono-*L*-tartrate of the compound of formula I.
Fig. 13 is the DSC pattern of the crystal form D of the mono-*L*-tartrate of the compound of formula I.
Fig. 14 is the TGA pattern of the crystal form D of the mono-*L*-tartrate of the compound of formula I.
Fig. 15 is the XRPD pattern of the crystal form A of the mono-*L*-tartrate of the compound of formula I in example 7.
Fig. 16 is the XRPD pattern of the crystal form B of the mono-*L*-tartrate of the compound of formula I in example 9.
Fig. 17 is the XRPD pattern of the crystal form A of the mono-*L*-tartrate of the compound of formula I after being placed under high temperature and high humidity conditions for 3 days.
Fig. 18 is the XRPD pattern of the crystal form A of the mono-*L*-tartrate of the compound of formula I after being placed under high temperature and high humidity conditions for 2 weeks.
Fig. 19 is the single crystal pattern of the mono-*L*-tartrate of the compound of formula I.

DETAILED DESCRIPTION

The technical solutions of the present disclosure will be further described in detail below in conjunction with specific examples. It should be understood that the following examples are only used to illustrate and explain the present disclosure, and should not be construed as a limitation on the scope of protection of the present disclosure. All techniques realized based on the above contents of the present disclosure are within the scope of protection intended by the present disclosure.

Unless otherwise stated, the raw materials and reagents used in the following examples are commercially available or can be prepared by known methods.

40°C/75% RH-open refers to being placed in an open environment at 40°C and 75% humidity.

60°C-open refers to being placed in an open environment at 60°C.

80% RH-open refers to being placed in an open environment at 80% humidity.

92.5% RH-open refers to being placed in an open environment at 80% humidity.

40°C/75% RH-closed-2wks refers to being placed in a closed environment for 2 weeks at 40°C and 75% humidity.

40°C/75% RH-open-2wks refers to being placed in an open environment for 2 weeks at 40°C and 75% humidity.

60°C-closed-2wks refers to being placed in a closed environment for 2 weeks at 60°C.

STD-1 refers to the control sample.

Initial refers to the initial state.

SGF refers to simulated gastric fluid.

FaSSIF refers to fasted state simulated intestinal fluid

FeSSIF refers to fed state simulated intestinal fluid.

1d refers to 1 day; 3d refers to 3 days.

**Parameters of experimental instruments**

**X-ray powder diffraction (XRPD)**

The equipment is Shimadzu XRD-6000, and the sample is scanned according to the following parameters:

The ray source is Cu-Kα target (1.54056 Å).

The minimum operating voltage and current of the light tube are 40 kV and 30 mA, respectively.

The 2-Theta values for the sample scanning range is from 2° to 50°. The scanning speed is 5 deg/minute.

**Thermogravimetric analysis (TGA)**

Approximately 5 mg of the sample was weighed into a crucible, heated from 30°C to 300°C at a heating rate of 20°C/minute under a nitrogen atmosphere, and held at 300°C for 1 minute.

**Differential scanning calorimeter (DSC)**

Approximately 1 to 5 mg of the powder sample was weighed into a closed aluminum crucible with a pinhole punched in the crucible lid. The sample was heated from 30°C to 300°C under a nitrogen atmosphere for differential calorimetry scanning, and held at 300°C for 1 minute. The heating rate was 20°C/minute.

**Polarized light microscopy (PLM)**

The sample was dispersed in a medium (silicone oil) and observed using a 10X eyepiece and a 10X objective lens, and the image was recorded with a camera computer system.

**Dynamic vapor sorption (DVS)**

Approximately 10 mg of the sample was weighed at 25°C under a 0% to 95% to 0% relative humidity (RH) cycle for testing the moisture absorption/desorption characteristics. The parameters are as follows:

| Setting | Parameters |
|---|---|
| Sample chamber temperature | 25°C |
| Equilibrium condition | dm/dt: 0.01%/min |
| Humidity range, RH (%) | 0% to 95% to 0% RH |
| Step size measurement, RH (%) | 5% RH |
| Sample amount | 10 to 20 mg |

Classification of hygroscopicity:

| Grades of hygroscopicity | Vapor sorption standard* |
|---|---|
| Deliquescence | Absorption of sufficient water to form a liquid |
| Highly hygroscopic | W ≥ 15% |

(continued)

| Grades of hygroscopicity | Vapor sorption standard* |
|---|---|
| Hygroscopic | 2% ≤ W < 15% |
| Slightly hygroscopic | 0.2% ≤ W < 2% |
| Non-hygroscopic | W < 0.2% |

"*": at 25 ± 1°C and 80 ± 2% RH (European Pharmacopoeia 10.0)
"W": hygroscopic weight gain at 80% RH.

**Single crystal test instrument and test conditions:**

**Instrument model: D8 Venture**

**Instrument parameters:**

| | |
|---|---|
| Light source: Cu target | X-ray: Cu-Kα (= 1.54 Å) |
| Detector: CMOS area detector | Resolution: 0.80 Å |
| Current and voltage: 50 kV, 1.2 mA | Exposure time: 50 s |
| Distance from area detector to sample: 40 mm | Test temperature: 170(2) K |

**Preparation of intermediate 1f and compound of formula I**

**(1) Preparation of intermediate 1f**

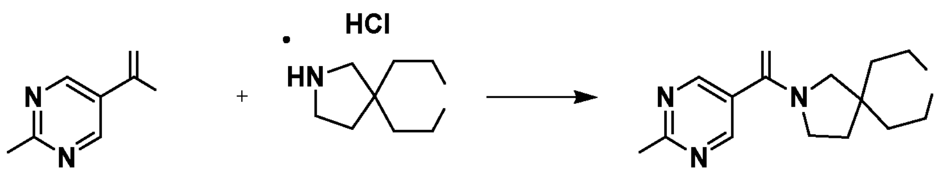

2-Chloropyrimidine-5-carboxylic acid (284 g, 1.78 mol) and 8-oxa-2-azaspiro[4.5]decane hydrochloride (310 g, 1.78 mol) were dissolved in dichloromethane. The reaction mixture was cooled to -10°C, and $T_3P$ (625 g, 1.78 mol) was slowly added dropwise thereto. After the addition was completed, the reaction was continued at the same temperature for 2 hours. After the completion of the reaction was detected by LCMS, the reaction mixture was added with water, stirred to precipitate a solid, filtered, and dried to get **compound 1f** (350 g, purity: 98%).

$^1$H NMR (400 MHz, $CDCl_3$): δ 8.80 (s, 2H), 3.81 - 3.57 (m, 7H), 3.36 (s, 1H), 1.93 (td, *J* = 14.58, 7.25 Hz, 2H), 1.66 (t, *J* = 5.35 Hz, 2H), 1.58 (dd, *J* = 11.10, 4.64 Hz, 2H).

**(2) Preparation of compound of formula I**

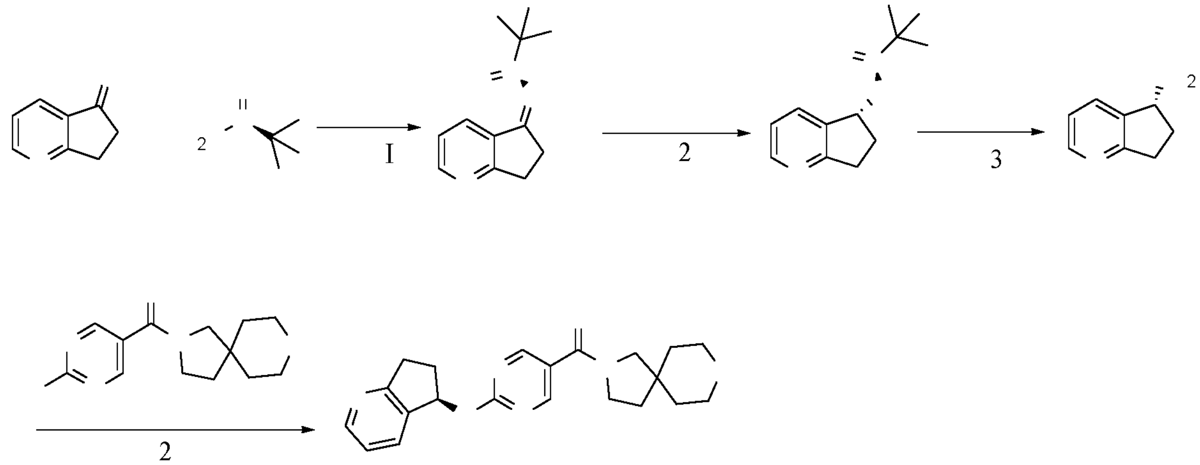


Step 1

Compound **1b** (385 g, 3.18 mol) and tetraethyl titanate (905 g, 3.97 mol) were dissolved in toluene (3 L). The reaction mixture was stirred, heated to 110°C, and refluxed, and a solution of compound **1a** (352 g, 2.65 mol) in toluene (500 mL) was added dropwise thereto. After the dropwise addition was completed, the reaction mixture was heated to reflux for another 1 hour. After the reaction was completed, the reaction was terminated. The reaction mixture was cooled to room temperature and concentrated to remove toluene. The residue was quenched with water (400 mL), diluted with ethyl acetate (1000 mL), and filtered through diatomite. The filtrate was subjected to phase separation. The organic phase was collected, dried over anhydrous sodium sulfate, and concentrated to remove the solvent. The residue was stirred with MTBE (100 mL) and petroleum ether (300 mL), filtered, and the filtrate was concentrated to get **compound 1c** (500 g, yield: 80%).

Step 2

**Compound 1c** (500 g, 2.1 mol) was dissolved in THF (3000 mL). The reaction mixture was cooled to -60°C, and then L-selectride (2510 mL, 2.51 mol) was slowly added dropwise thereto. After the dropwise addition was completed, the reaction mixture was stirred at -60°C. After the reaction was completed, the reaction was quenched by slowly adding water (1000 mL). The reaction mixture was concentrated to remove most of the THF. The reaction mixture was extracted with ethyl acetate (500 mL × 3). The organic phases were combined, washed with saturated sodium chloride solution (200 mL), and dried. The organic phase was distilled under reduced pressure to remove the solvent, and the crude product **1d** was directly used in the next step.

Step 3

The crude product **1d** was dissolved in methanol (500 mL), and HCl/MeOH (200 mL, 4 M) was slowly added thereto. The reaction mixture was reacted at room temperature for 2 hours. After the reaction was completed, the reaction mixture was concentrated to obtain an oil, stirred with ethyl acetate (100 mL), and filtered to get compound 1e as a red solid powder (300 g, ee value: 98%, purity: 99%).

Step 4

**Compound 1e** (284 g, 1.375 mol), **compound 1f** (350 g, 1.25 mol), and $K_2CO_3$ (862.5 g, 6.25 mol) were dissolved in isopropanol (400 mL), and the reaction mixture was heated to reflux overnight. After the reaction was completed, the reaction mixture was cooled, filtered, and the filtrate was concentrated. The residue was dissolved in water (300 mL), added with dilute hydrochloric acid (2 N) to adjust the pH 8 to 9, extracted with dichloromethane (200 mL × 3), washed with saturated sodium chloride solution (200 mL × 1), dried, and concentrated to get the **compound of formula I** (374 g).

$^1$H NMR (400 MHz, $CD_3OD$) δ 8.58 (s, 2H), 8.37 (d, *J* = 5.1 Hz, 1H), 7.77 (s, 1H), 7.26 (d, *J* = 2.5 Hz, 1H), 5.72 (t, *J* = 7.7 Hz, 1H), 4.53 (s, 2H), 3.80 - 3.58 (m, 7H), 3.54 (d, *J* = 18.8 Hz, 2H), 3.15 (ddd, *J* = 16.9, 9.2, 3.7 Hz, 1H), 3.02 (dt, *J* = 16.8, 8.5 Hz, 1H), 2.68 (dq, *J* = 12.8, 4.4 Hz, 1H), 2.14 - 2.02 (m, 1H), 1.93 (q, *J* = 8.1 Hz, 2H), 1.67 (d, *J* = 5.8 Hz, 2H), 1.59 (d, *J* = 5.7 Hz, 2H).

**Example 1: Preparation of mono-*L*-tartrate of compound of formula I**

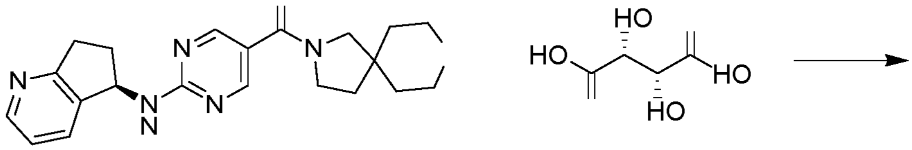

I

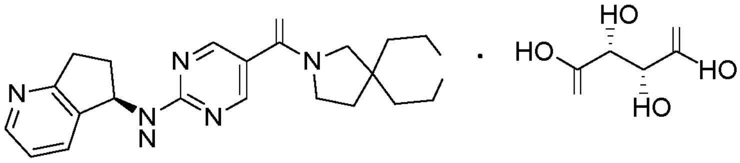

I-1

1 g of the compound of formula I was dissolved in 18 mL of acetone, then 1.24 mL of 2 mol/L ethanol solution of *L*-tartaric acid was added thereto, and the resulting mixture was stirred overnight. The mixture was then added with 18 mL of isopropyl acetate to obtain a solid, filtered, and vacuum-dried under reduced pressure to obtain an *L*-tartrate of the compound of formula I (the molar ratio of the compound of formula I to *L*-tartaric acid was 1: 1, *i.e.,* a mono-*L*-tartrate was obtained).

50 mg of the *L*-tartrate of the compound of formula I was transferred to an 8 mL glass vial and dissolved in 1 mL of methanol by ultrasonication. The resulting mixture was filtered, and the filtrate was transferred to a new 8 mL glass vial. The 8 mL open glass vial was placed in a 40 mL glass vial containing 4 mL of methyl *tert*-butyl ether with the cap tightened, which was placed for single crystal cultivation. The single crystal data of the mono-*L*-tartrate of the compound of formula I are as follows:

| | |
|---|---|
| $C_{21}H_{25}N_5O_2 \cdot C_4H_6O_6$ | F(000) = 560 |
| $M_r$ = 529.55 | $D_x$ = 1.273 Mg $m^{-3}$ |
| Monoclinic, $P2_1$ | Cu $K\alpha$ radiation, $\lambda$ = 1.54178 Å |
| *a* = 6.3430 (5) Å | Cell parameters from 712 reflections |
| *b* = 8.8372 (7) Å | $\theta$ = 5.3 - 71.6° |
| c = 24.809 (2) Å | $\mu$ = 0.81 $mm^{-1}$ |
| $\beta$ = 96.577 (5)° | *T*= 170 K |
| *V* = 1381.5 (2) $Å^3$ | Plate, colourless |
| Z=2 | 0.08 × 0.05 × 0.03 mm |

The single crystal of the mono-*L*-tartrate of the compound of formula I is shown in Fig. 19.

**Example 2: Preparation of fumarate of compound of formula I**

536 mg of the compound of formula I was dissolved in 10 mL of acetone, then 161.4 mg of fumaric acid was added thereto, and the resulting mixture was stirred overnight. The mixture was then added with 10 mL of isopropyl acetate, stirred to obtain a solid, filtered, and vacuum-dried under reduced pressure to obtain a fumarate of the compound of formula I.

**Example 3: Preparation of D-malate of compound of formula I**

517 mg of the compound of formula I was dissolved in 10 mL of isopropyl acetate, then 187.4 mg of *D*-malic acid was added thereto, and the resulting mixture was stirred to become viscous. The mixture was then added with 10 mL of isopropyl acetate to obtain a white viscous solid, filtered, and vacuum-dried under reduced pressure to obtain a *D*-malate

of the compound of formula I.

**Example 4: Stability test of mono-L-tartrate, fumarate, and D-malate of compound of formula I**

The stability of the mono-*L*-tartrate, fumarate, and *D*-malate of the compound of formula I obtained in examples 1 to 3 was studied.

Stability study conditions: 40°C/75% RH-closed, 40°C/75% RH-open, 60°C-closed; Stability study content: changes in related substances and crystal forms.

Detection of related substances: Approximately 5 mg of the sample was weighed into a 40 mL sample vial, dissolved in 10 mL of 50% acetonitrile aqueous solution and diluted to the scale, and 5 μL of the sample was injected. The chromatographic conditions are shown in Table 5.

The experimental results of the stability study on the mono-L-tartrate, fumarate, and D-malate of the compound of formula I are shown in Table 6.

**Table 5: Chromatographic conditions for testing of related substances**

<table>
<tr><td>Liquid chromatography equipment</td><td>Agilent 1260</td></tr>
<tr><td>Chromatographic column</td><td>Agilent Eclipse Plus C18 (4.6 * 250 mm, 5 μm)</td></tr>
<tr><td>Mobile phase</td><td>A: 0.1% TFA aqueous solution; B: 0.1% TFA acetonitrile solution</td></tr>
<tr><td>Gradient elution</td><td><table>
<tr><td>Time</td><td>0</td><td>1</td><td>23</td><td>25</td><td>25.5</td><td>30</td></tr>
<tr><td>A%</td><td>90</td><td>90</td><td>10</td><td>10</td><td>90</td><td>90</td></tr>
<tr><td>B%</td><td>10</td><td>10</td><td>90</td><td>90</td><td>10</td><td>10</td></tr>
</table></td></tr>
<tr><td>Detection wavelength (nm)</td><td>270</td></tr>
<tr><td>Injection volume (μL)</td><td>5</td></tr>
<tr><td>Sample concentration (mg/mL)</td><td>0.5</td></tr>
<tr><td>Column temperature (°C)</td><td>25</td></tr>
<tr><td>Flow rate (mL/min)</td><td>1.0</td></tr>
<tr><td>Diluent</td><td>50% acetonitrile aqueous solution</td></tr>
<tr><td>Run time (min)</td><td>30</td></tr>
</table>

Table 6: Experimental results of stability study on mono-L-tartrate, fumarate, and D-malate of compound of formula I

<table>
<tr><th rowspan="2">Salt form</th><th rowspan="2">Stability conditions</th><th rowspan="2">Total impurities %</th><th>8.00</th></tr>
<tr><th>1.00</th></tr>
<tr><td rowspan="4">Mono-L-tartrate of compound of formula I</td><td>Initial</td><td>0.06</td><td>99.94</td></tr>
<tr><td>40°C/75% RH-closed-2wks</td><td>0.06</td><td>99.94</td></tr>
<tr><td>40°C/75% RH-open-2wks</td><td>0.10</td><td>99.90</td></tr>
<tr><td>60°C-open-2wks</td><td>0.08</td><td>99.92</td></tr>
<tr><td rowspan="4">Fumarate of compound of formula I</td><td>Initial</td><td>0.31</td><td>99.69</td></tr>
<tr><td>40°C/75% RH-closed-2wks</td><td>0.30</td><td>99.70</td></tr>
<tr><td>40°C/75% RH-open-2wks</td><td>0.31</td><td>99.69</td></tr>
<tr><td>60°C-open-2wks</td><td>0.32</td><td>99.68</td></tr>
</table>

(continued)

| Salt form | Stability conditions | Total impurities % | 8.00 |
|---|---|---|---|
| | | | 1.00 |
| D-malate of **compound of formula I** | Initial | 0.11 | 99.89 |
| | 40°C/75% RH-closed-20d | 0.09 | 99.91 |
| | 40°C/75% RH-open-20d | 0.10 | 99.90 |
| | 60°C-open-20d | 0.18 | 99.82 |

As can be seen from Table 6, various salt forms, *i.e.,* the L-tartrate, fumarate, and *D*-malate of the compound of formula I, still had good chemical stability after accelerated high humidity and high temperature testing, especially for the L-tartrate of the compound of formula I.

**Example 5: Solubility test of compound of formula I and L-tartrate, fumarate, and D-malate thereof**

The solubility of the compound of formula I and the L-tartrate, fumarate, and D-malate thereof was studied in water, SGF, FaSSIF, FeSSIF, and pH 7.4 at 37°C.

Experimental method: 30 mg (in water) or 15 mg of the sample was weighed into a 4 mL vial, then 3 mL of the medium to be tested was added thereto, and the resulting mixture was stirred continuously at 37°C. 0.5 mL of the sample was collected at 1 hour and 24 hours, respectively, and centrifuged at 12,000 rpm for 5 minutes. The supernatant was diluted to an appropriate multiple with 50% acetonitrile and its concentration was determined. The chromatographic conditions for the solubility test are shown in Table 7.

Control and linearity: The compound of formula I is not suitable to be used as a control as it is highly hygroscopic and has too many impurities. Therefore, 13 mg of the fumarate of the compound of formula I was weighed into a 25 mL volumetric flask (approximately 10 mg added to the 25 mL volumetric flask, calculated as free base), dissolved in 50% acetonitrile aqueous solution and diluted to the scale, and two samples were prepared in parallel. STD-1 was diluted to 200 μg/mL, 50 μg/mL, and 10 μg/mL with 50% acetonitrile aqueous solution. 5 μL of the sample was injected, and the standard curve was plotted.

The solubility test results of the compound of formula I and the *L*-tartrate, fumarate, and *D*-malate thereof are shown in Table 8.

**Table 7: Chromatographic conditions for solubility test**

| | |
|---|---|
| Liquid chromatography equipment | Agilent 1260 |
| Chromatographic column | Waters Sunfire C18 (4.6 * 150 mm, 5 μm) |
| Mobile phase | A: pH 7.8 sodium dihydrogen phosphate buffer solution (7.2 g of sodium phosphate dibasic dodecahydrate to 1 L of water, added with phosphoric acid to adjust the pH to 7.8); B: acetonitrile |
| Isocratic elution | A: B = 65:35 |
| Detection wavelength (nm) | 270 |
| Injection volume (μL) | 5 |
| Column temperature (°C) | 40 |
| Flow rate (mL/min) | 1.0 |
| Diluent | 50% acetonitrile aqueous solution |
| Run time (min) | 5 |

**Table 8: Solubility test results of the compound of formula I and the *L*-tartrate, fumarate, and *D*-malate thereof**

| Sample | Medium | Sample amount (mg) | Medium volume (mL) | Solubility (mg/mL) (1 h) | Solubility (mg/mL) (24 h) | Sample appearance | Final pH |
|---|---|---|---|---|---|---|---|
| Compound of formula I | Water | 31.49 | 3 | 10.62 | 10.84 | Clarified solution | 7.32 |
| | SGF | 15.81 | 3 | 5.23 | 5.39 | Clarified solution | 1.37 |
| | FaSSIF | 15.32 | 3 | 5.10 | 5.22 | Clarified solution | 6.38 |
| | FeSSIF | 15.47 | 3 | 5.46 | 5.33 | Clarified solution | 4.99 |
| | pH7.4 | 15.43 | 3 | 5.11 | 5.20 | Clarified solution | 7.56 |
| Mono-*L*-tartrate of compound of formula I | Water | 42.92 | 3 | 10.32 | 10.48 | Clarified solution | 3.21 |
| | SGF | 21.76 | 3 | 5.19 | 5.25 | Clarified solution | 1.37 |
| | FaSSIF | 20.69 | 3 | 5.08 | 5.12 | Clarified solution | 3.84 |
| | FeSSIF | 20.82 | 3 | 5.12 | 5.02 | Clarified solution | 4.69 |
| | pH7.4 | 20.65 | 3 | 5.09 | 4.97 | Clarified solution | 6.58 |
| Fumarate of compound of formula I | Water | 40.99 | 3 | 11.05 | 11.37 | Clarified solution | 3.32 |
| | SGF | 20.05 | 3 | 5.37 | 5.46 | Clarified solution | 1.36 |
| | FaSSIF | 20.28 | 3 | 5.44 | 5.57 | Clarified solution | 4.01 |
| | FeSSIF | 21.22 | 3 | 5.75 | 5.63 | Clarified solution | 4.71 |
| | pH7.4 | 20.87 | 3 | 5.65 | 5.61 | Clarified solution | 6.54 |
| *D*-malate of compound of formula I | Water | 41.20 | 3 | 10.60 | 10.76 | Clarified solution | 3.75 |
| | SGF | 20.72 | 3 | 5.36 | 5.37 | Clarified solution | 1.36 |
| | FaSSIF | 20.31 | 3 | 5.26 | 5.36 | Clarified solution | 4.47 |
| | FeSSIF | 20.57 | 3 | 5.31 | 5.28 | Clarified solution | 4.75 |
| | pH7.4 | 21.58 | 3 | 5.54 | 5.53 | Clarified solution | 6.60 |

As can be seen from Table 8, the compound of formula I and the *L*-tartrate, fumarate, and *D*-malate thereof all have good solubility. Each medium allows for the complete dissolution to a target concentration of 5 mg/mL or 10 mg/mL, calculated as free base.

**Example 6: Preparation of crystal form A**

To 400 mg of the mono-*L*-tartrate of the compound of formula I (prepared according to example 1) was added 10 mL of acetone. The resulting mixture was stirred, centrifuged, and vacuum-dried under reduced pressure at 40°C to obtain a crystal form A.

The crystal form A was characterized by XRPD, DSC, TGA, and PLM.

The crystal form A is an anhydride. The XRPD characteristic peak positions and intensities are shown in Table 1, and the XRPD pattern is shown in Fig. 1.

DSC shows a first endothermic peak near the peak temperature of 150.14°C when heated, as shown in Fig. 2.

TGA shows a weight loss of approximately 0.069% in the range of 22.03°C to 120°C, as shown in Fig. 3.

The PLM pattern shows that the sample is a crystal with irregular morphology of 10 μm or less, as shown in Fig. 4.

In the X-ray powder diffraction pattern of the crystal form A expressed at 2θ angles, the 2θ values are shown in Table A.

Table A: XRPD analysis data of crystal form A

| Peak number | 2θ [°] | Relative intensity% | Peak number | 2θ [°] | Relative intensity% |
|---|---|---|---|---|---|
| 1 | 8.30 | 28.3 | 17 | 26.90 | 10.2 |
| 2 | 10.22 | 11.9 | 18 | 28.44 | 11.9 |

(continued)

| Peak number | 2θ [°] | Relative intensity% | Peak number | 2θ [°] | Relative intensity% |
|---|---|---|---|---|---|
| 3 | 12.44 | 8 | 19 | 28.92 | 19.4 |
| 4 | 14.24 | 24.2 | 20 | 30.30 | 9.1 |
| 5 | 15.60 | 20.4 | 21 | 31.10 | 8.4 |
| 6 | 17.06 | 70.8 | 22 | 32.52 | 17.2 |
| 7 | 18.00 | 54.4 | 23 | 34.60 | 15.7 |
| 8 | 18.80 | 60 | 24 | 35.16 | 13.4 |
| 9 | 19.22 | 51 | 25 | 36.28 | 10.7 |
| 10 | 20.06 | 95.8 | 26 | 38.12 | 11 |
| 11 | 20.52 | 32.6 | 27 | 39.28 | 8.2 |
| 12 | 22.58 | 100 | 28 | 39.88 | 5.6 |
| 13 | 23.72 | 63.4 | 29 | 40.90 | 8.7 |
| 14 | 24.38 | 55.2 | 30 | 41.54 | 4.8 |
| 15 | 25.32 | 18.7 | 31 | 45.52 | 6.2 |
| 16 | 25.70 | 31.1 | 32 | 49.06 | 4.4 |

**Example 7: Preparation of crystal form A**

To 500 mg of the mono-L-tartrate of the compound of formula I (prepared according to example 1) was added 10 mL of methanol for dissolution, and then 60 mL of methyl *tert*-butyl ether was added thereto. The resulting mixture was stirred for 1.5 hours, centrifuged, filtered, and dried to obtain the crystal form A.

The XRPD pattern of the crystal form A is shown in Fig. 15.

**Example 8: Preparation of crystal form B**

500 mg of the crystal form A of the mono-L-tartrate of the compound of formula I was exposed to 92.5% RH for 3 days to obtain a crystal form B.

The crystal form B was characterized by XRPD, DSC, TGA, and PLM.

The crystal form B is a hydrate, which contains 0.5 to 1 mol of water. The XRPD characteristic peak positions and intensities are shown in Table B, and the XRPD pattern is shown in Fig. 5.

DSC shows a first endothermic peak near the peak temperature of 61.57°C when heated and a second endothermic peak near the peak temperature of 152.18°C when heated, as shown in Fig. 6.

TGA shows a weight loss of approximately 2.76% in the range of 22.03°C to 120°C, as shown in Fig. 7.

In the X-ray powder diffraction pattern of the crystal form B expressed at 2θ angles, the 2θ values are shown in Table B.

Table B: XRPD analysis data of crystal form B

| Peak number | 2θ [°] | Relative intensity% | Peak number | 2θ [°] | Relative intensity% |
|---|---|---|---|---|---|
| 1 | 3.78 | 23 | 21 | 26.72 | 22 |
| 2 | 7.56 | 58.2 | 22 | 28.56 | 15.6 |
| 3 | 10.62 | 7.7 | 23 | 29.52 | 37.9 |
| 4 | 14.16 | 16.5 | 24 | 30.66 | 16.5 |
| 5 | 15.06 | 19.6 | 25 | 31.20 | 15.4 |
| 6 | 15.62 | 14.1 | 26 | 33.16 | 17.5 |
| 7 | 16.48 | 22.4 | 27 | 33.78 | 17 |
| 8 | 17.36 | 60.1 | 28 | 34.58 | 12.7 |

(continued)

| Peak number | 2θ [°] | Relative intensity% | Peak number | 2θ [°] | Relative intensity% |
|---|---|---|---|---|---|
| 9 | 18.14 | 27.2 | 29 | 35.12 | 20.4 |
| 10 | 18.58 | 21.6 | 30 | 35.60 | 8.6 |
| 11 | 19.28 | 100 | 31 | 36.42 | 7.9 |
| 12 | 19.94 | 98.2 | 32 | 38.20 | 11.3 |
| 13 | 21.30 | 72.9 | 33 | 39.60 | 4 |
| 14 | 22.56 | 16 | 34 | 40.36 | 7.3 |
| 15 | 22.90 | 4.6 | 35 | 41.02 | 3.5 |
| 16 | 23.72 | 70.9 | 36 | 42.20 | 5.8 |
| 17 | 24.52 | 28 | 37 | 43.98 | 8.5 |
| 18 | 25.00 | 14 | 38 | 45.08 | 6.7 |
| 19 | 25.42 | 11.3 | 39 | 47.32 | 6.1 |
| 20 | 26.02 | 51.3 | | | |

**Example 9: Preparation of crystal form B**

To 414 mg of the mono-L-tartrate of the compound of formula I (prepared according to example 1) was added 10 mL of methanol for complete dissolution. The solvent was naturally evaporated to obtain a white solid, which was dried under reduced pressure to obtain a crystal form B.

The XRPD pattern of the crystal form B is shown in Fig. 16.

**Example 10: Preparation of crystal form C**

To 410 mg of the mono-L-tartrate of the compound of formula I (prepared according to example 1) was added 16 mL of ethanol. The resulting mixture was stirred at 50°C for 1 day and cooled to room temperature to precipitate a white solid, which was filtered and vacuum-dried under reduced pressure to obtain a crystal form C.

The crystal form C was characterized by XRPD, DSC, TGA, and PLM.

The crystal form C is an ethanol solvate.

The XRPD characteristic peak positions and intensities are shown in Table C, and the XRPD pattern is shown in Fig. 8.

DSC shows a first endothermic peak near the peak temperature of 129.45°C when heated, as shown in Fig. 9.

TGA shows a weight loss of approximately 4.59% in the range of 21.47°C to 150°C, as shown in Fig. 10.

In the X-ray powder diffraction pattern of the crystal form C expressed at 2θ angles, the 2θ values are shown in Table C.

Table C: XRPD analysis data of crystal form C

| Peak number | 2θ [°] | Relative intensity% | Peak number | 2θ [°] | Relative intensity% |
|---|---|---|---|---|---|
| 1 | 3.58 | 28.6 | 13 | 24.38 | 12.5 |
| 2 | 7.14 | 51.6 | 14 | 25.00 | 7.7 |
| 3 | 10.46 | 17.3 | 15 | 25.92 | 36.6 |
| 4 | 12.22 | 9.2 | 16 | 27.58 | 9.1 |
| 5 | 13.96 | 26.5 | 17 | 28.06 | 11 |
| 6 | 14.56 | 17.5 | 18 | 29.38 | 21 |
| 7 | 17.10 | 35 | 19 | 30.22 | 5.1 |
| 8 | 17.88 | 77.1 | 20 | 30.78 | 12.3 |
| 9 | 19.40 | 75 | 21 | 33.66 | 9.1 |

(continued)

| Peak number | 2θ [°] | Relative intensity% | Peak number | 2θ [°] | Relative intensity% |
|---|---|---|---|---|---|
| 10 | 20.06 | 48.8 | 22 | 35.24 | 8.9 |
| 11 | 21.38 | 100 | 23 | 37.40 | 5.6 |
| 12 | 23.76 | 49.1 | 24 | 38.86 | 4.2 |

The PLM pattern shows that the sample is a crystal with irregular morphology of 10 μm or less, as shown in Fig. 11.

**Example 11: Preparation of crystal form D**

To 410 mg of the mono-L-tartrate of the compound of formula I (prepared according to example 1) was added 12 mL of tetrahydrofuran. The resulting mixture was stirred at 50°C for 1 day and cooled to room temperature to precipitate a white solid, which was filtered and vacuum-dried under reduced pressure to obtain a crystal form D.

The crystal form D was characterized by XRPD, DSC, and TGA.

The crystal form D is a tetrahydrofuran solvate.

The XRPD characteristic peak positions and intensities are shown in Table D, and the XRPD pattern is shown in Fig. 12.

DSC shows a first endothermic peak near the peak temperature of 94.15°C when heated, as shown in Fig. 13.

TGA shows a weight loss of approximately 5.25% in the range of 23.19°C to 120°C, as shown in Fig. 14.

In the X-ray powder diffraction pattern of the crystal form D expressed at 2θ angles, the 2θ values are shown in Table D.

Table D: XRPD analysis data of crystal form D

| Peak number | 2θ [°] | Relative intensity% | Peak number | 2θ [°] | Relative intensity% |
|---|---|---|---|---|---|
| 1 | 3.50 | 87.2 | 10 | 21.88 | 17.8 |
| 2 | 6.92 | 71.3 | 11 | 23.04 | 100 |
| 3 | 7.46 | 78.1 | 12 | 24.50 | 18.8 |
| 4 | 10.38 | 15.7 | 13 | 25.62 | 37.3 |
| 5 | 15.06 | 17.5 | 14 | 26.50 | 14.1 |
| 6 | 17.22 | 71.8 | 15 | 28.22 | 12 |
| 7 | 18.20 | 55.6 | 16 | 28.86 | 12 |
| 8 | 19.88 | 53.8 | 17 | 30.28 | 19.6 |
| 9 | 20.76 | 31.9 | 18 | 32.08 | 19.1 |

**Example 12: Stability study on crystal form A**

The crystal form A was examined whether the crystal form had changed after exposure to 40°C/75% RH-open, 60°C-open, 80% RH-open, and 92.5% RH-open for 3 days.

In addition, samples were examined for their stability after exposure to 40°C/75% RH-closed, 40°C/75% RH-open, and 60°C-open for 2 weeks, including related substances and crystal forms.

Detection method of stability sample related substances: Approximately 5 mg of the sample was weighed, added with 5 mL of 20% methanol, ultrasonicated for a few seconds so that the compound can be completely dissolved, and 5 μL of the sample was injected for testing of related substances.

The XRPD patterns of the crystal form A after being placed under high temperature and high humidity conditions for 3 days and 2 weeks are shown in Fig. 17 and Fig. 18, respectively.

Chromatographic conditions:

<table>
<tr><td>Chromatographic column:</td><td colspan="3">Agilent zorbax bonus-RP 4.6 * 150 mm, 3.5 μm</td></tr>
<tr><td>Mobile phase:</td><td colspan="3">A: 0.05% TFA aqueous solution; B: 0.05% TFA acetonitrile solution</td></tr>
<tr><td rowspan="8">Gradient:</td><td>Time (minute)</td><td>Mobile phase A (%)</td><td>Mobile phase B (%)</td></tr>
<tr><td>0.0</td><td>100</td><td>0</td></tr>
<tr><td>3.0</td><td>100</td><td>0</td></tr>
<tr><td>15</td><td>50</td><td>50</td></tr>
<tr><td>16</td><td>10</td><td>90</td></tr>
<tr><td>19</td><td>10</td><td>90</td></tr>
<tr><td>19.1</td><td>100</td><td>0</td></tr>
<tr><td>26</td><td>100</td><td>0</td></tr>
<tr><td>Column temperature:</td><td colspan="3">30°C</td></tr>
<tr><td>Flow rate:</td><td colspan="3">0.8 mL/min</td></tr>
<tr><td>Injection volume:</td><td colspan="3">5 μL</td></tr>
<tr><td>Detection wavelength:</td><td colspan="3">270 nm</td></tr>
<tr><td>Run time:</td><td colspan="3">26 min</td></tr>
</table>

**Table 9: Stability test results of crystal form A**

| Sample | Weight (mg) | Volume (mL) | Total impurities% | Individual impurities% | Purity% |
|---|---|---|---|---|---|
| Initial | 5.14 | 5 | 0.08 | 0.04 | 99.92 |
| 40°C/75% RH-closed-2wks | 5.28 | 5 | 0.08 | 0.04 | 99.92 |
| 40°C/75% RH-open-2wks | 5.66 | 5 | 0.11 | 0.06 | 99.89 |
| 60°C-open-2wks | 5.58 | 5 | 0.13 | 0.06 | 99.87 |
| 80% RH-open-2wks | 5.25 | 5 | 0.10 | 0.05 | 99.90 |
| 92.5% RH-open-2wks | 5.33 | 5 | 0.11 | 0.06 | 99.89 |

As shown in Table 9, Fig. 17, and Fig. 18, the crystal form A has not changed at 60°C under high humidity conditions of 80% RH. This indicates that the crystal form A has good stability.

**Example 13: Solubility study on crystal form A**

The solubility of the crystal form Ain SGF, FaSSIF, FeSSIF, and pH 7.4 was evaluated.

20 mg of raw material was weighed into a vial, then 3 mL of medium was added thereto, and the dissolution was observed at 37°C.

**Table 10: Solubility test results of the crystal form A of the mono-L-tartrate of the compound of formula I (37°C)**

| Sample | Medium | Sample amount (mg) | Medium volume (mL) | Phenomenon | Solubility (mg/mL) | Final pH |
|---|---|---|---|---|---|---|
| Crystal form A | SGF | 21.25 | 3 | Solid dissolves immediately to form a solution | > 7.1 | 1.29 |
| | FaSSIF | 22.90 | 3 | Solid dissolves immediately to form a solution | > 7.6 | 4.32 |
| | FeSSIF | 21.87 | 3 | Solid dissolves immediately to form a solution | > 7.3 | 4.77 |
| | pH 7.4 | 21.73 | 3 | Solid dissolves immediately to form a solution | > 7.2 | 6.57 |

As can be seen from Table 10, the crystal form A has good solubility in all media.

The specific examples of the present disclosure have been described above. It should be understood that the present disclosure is not limited to the foregoing examples, and the foregoing examples and the descriptions in the specification are only for the purpose of illustrating the principles of the present disclosure. Various non-substantial modifications and improvements can be made to the present disclosure by those skilled in the art without departing from the conception of the present disclosure, all of which fall within the scope of protection of the present disclosure.

## Claims

1. A pharmaceutically acceptable salt of a compound of formula I, wherein the compound of formula I is as follows:

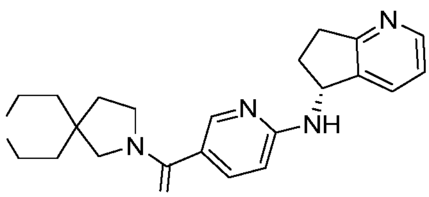

I

the pharmaceutically acceptable salt of the compound of formula I is a salt formed by the compound of formula I with an acid.

2. The pharmaceutically acceptable salt of the compound of formula I according to claim 1, wherein the acid is selected from an inorganic acid or an organic acid, such as hydrochloric acid, hydrofluoric acid, hydrobromic acid, hydroiodic acid, sulfuric acid, pyrosulfuric acid, phosphoric acid, nitric acid, formic acid, acetic acid, acetoacetic acid, pyruvic acid, trifluoroacetic acid, propionic acid, butyric acid, hexanoic acid, heptanoic acid, undecanoic acid, lauric acid, benzoic acid, salicylic acid, 2-(4-hydroxybenzoyl)benzoic acid, camphoric acid, cinnamic acid, cyclopentylpropionic acid, digluconic acid, 3-hydroxy-2-naphthoic acid, nicotinic acid, pamoic acid, pectic acid, persulfuric acid, 3-phenylpropionic acid, picric acid, pivalic acid, 2-hydroxyethanesulfonic acid, itaconic acid, sulfamic acid, trifluoromethanesulfonic acid, dodecyl sulfuric acid, ethanesulfonic acid, benzenesulfonic acid, p-toluenesulfonic acid, methanesulfonic acid, 2-naphthalenesulfonic acid, naphthalenedisulfonic acid, camphorsulfonic acid, citric acid, L-tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid, glucoheptanoic acid, glycerophosphoric acid, aspartic acid, sulfosalicylic acid, hemi-sulfuric acid, or thiocyanic acid.

3. The pharmaceutically acceptable salt of the compound of formula I according to claim 1, wherein the acid may be selected from one of hydrochloric acid, hydrobromic acid, sulfuric acid, phosphoric acid, nitric acid, methanesulfonic acid, p-toluenesulfonic acid, fumaric acid, maleic acid, citric acid, *L*-tartaric acid, succinic acid, ethanesulfonic acid, *L*-malic acid, *L*-glutamic acid, oxalic acid, *D*-malic acid, pamoic acid, oxalic acid, formic acid, acetic acid, trifluoroacetic acid, lauric acid, benzoic acid, and benzenesulfonic acid.

4. The pharmaceutically acceptable salt of the compound of formula I according to claim 1, wherein the molar ratio of the compound of formula I to the acid is 1:1; preferably, the pharmaceutically acceptable salt is selected from a monotartrate, a monofumarate, and a monomalate of the compound of formula I; more preferably, the pharmaceutically acceptable salt is a mono-*L*-tartrate of the compound of formula I.

5. A preparation method for the pharmaceutically acceptable salt of the compound of formula I according to claim 1, comprising reacting the compound of formula I with the acid to prepare the pharmaceutically acceptable salt of the compound of formula I;

   preferably, the preparation method comprises dissolving the compound of formula I in an organic solvent A, adding an acid for a reaction, and then adding an organic solvent B to prepare the pharmaceutically acceptable salt of the compound of formula I; the organic solvent A is selected from at least one of an ester, a ketone, and an alcohol; the organic solvent B is selected from a nitrile, an ester, an ether, or a combination thereof;
   preferably, the acid is first dissolved in an organic solvent C to prepare an acid solution form before being added to the reaction; more preferably, when the acid is selected from L-tartaric acid, the organic solvent C is selected from an alcohol.

6. A crystal form of the pharmaceutically acceptable salt of the compound of formula I according to claim 1, wherein the crystal form is a crystal form of a mono-L-tartrate of the compound of formula I.

7. The crystal form according to claim 6, wherein the crystal form is a crystal form A of the mono-L-tartrate of the compound of formula I, and the crystal form A has characteristic peaks at 17.06 ± 0.20°, 20.06 ± 0.20°, 22.58 ± 0.20° in an X-ray powder diffraction expressed at a 2θ angle using Cu-Kα radiation;

   preferably, the crystal form A has characteristic peaks at 17.06 ± 0.20°, 18.00 ± 0.20°, 18.80 ± 0.20°, 19.22 ± 0.20°, 20.06 ± 0.20°, 22.58 ± 0.20°, 23.72 ± 0.20°, 24.38 ± 0.20° in an X-ray powder diffraction expressed at a 2θ angle using Cu-Kα radiation;
   preferably, the crystal form A has characteristic peaks at 8.30 ± 0.20°, 14.24 ± 0.20°, 17.06 ± 0.20°, 18.00 ± 0.20°, 18.80 ± 0.20°, 19.22 ± 0.20°, 20.06 ± 0.20°, 20.52 ± 0.20°, 22.58 ± 0.20°, 23.72 ± 0.20°, 24.38 ± 0.20°, 25.70 ± 0.20° in an X-ray powder diffraction expressed at a 2θ angle using Cu-Kα radiation;
   preferably, the crystal form A has an X-ray powder diffraction expressed at a 2θ angle using Cu-Kα radiation as shown in Table 1, with an error range of ± 0.20°;
   preferably, the crystal form A has an X-ray powder diffraction pattern substantially as shown in Fig. 1;
   preferably, the crystal form A is an anhydride of the *L*-tartrate of the compound of formula I;
   preferably, the crystal form A has a first endothermic peak near a peak temperature of 150.14°C when heated, as shown by differential scanning calorimetry (DSC) analysis;
   preferably, the crystal form A has a DSC pattern substantially as shown in Fig. 2;
   preferably, the crystal form A has a weight loss of approximately 0.069% in a range of 22.03°C to 120°C, as shown by thermogravimetric analysis (TGA);
   preferably, the crystal form A has a TGA pattern substantially as shown in Fig. 3.

8. The crystal form according to claim 6, wherein the crystal form is a crystal form B of the mono-L-tartrate of the compound of formula I, and the crystal form B has characteristic peaks at 19.28 ± 0.20°, 19.94 ± 0.20°, 21.30 ± 0.20°, 23.72 ± 0.20° in an X-ray powder diffraction expressed at a 2θ angle using Cu-Kα radiation;

   preferably, the crystal form B has characteristic peaks at 7.56 ± 0.20°, 17.36 ± 0.20°, 19.28 ± 0.20°, 19.94 ± 0.20°, 21.30 ± 0.20°, 23.72 ± 0.20°, 26.02 ± 0.20° in an X-ray powder diffraction expressed at a 2θ angle using Cu-Kα radiation;
   preferably, the crystal form B has characteristic peaks at 7.56 ± 0.20°, 17.36 ± 0.20°, 18.14 ± 0.20°, 19.28 ± 0.20°, 19.94 ± 0.20°, 21.30 ± 0.20°, 23.72 ± 0.20°, 24.52 ± 0.20°, 26.02 ± 0.20°, 29.52 ± 0.20° in an X-ray powder diffraction expressed at a 2θ angle using Cu-Kα radiation;
   preferably, the crystal form B has characteristic peaks at 3.78 ± 0.20°, 7.56 ± 0.20°, 17.36 ± 0.20°, 18.14 ± 0.20°, 19.28 ± 0.20°, 19.94 ± 0.20°, 21.30 ± 0.20°, 23.72 ± 0.20°, 24.52 ± 0.20°, 26.02 ± 0.20°, 29.52 ± 0.20° in an X-ray powder diffraction expressed at a 2θ angle using Cu-Kα radiation;
   preferably, the crystal form B has an X-ray powder diffraction expressed at a 2θ angle using Cu-Kα radiation as shown in Table 2, with an error range of ± 0.20°;
   preferably, the crystal form B has an X-ray powder diffraction pattern substantially as shown in Fig. 5;
   the crystal form B is a hydrate of the *L*-tartrate of the compound of formula I;

the crystal form B has a first endothermic peak near a peak temperature of 61.57°C when heated and a second endothermic peak near a peak temperature of 152.18°C when heated, as shown by differential scanning calorimetry (DSC) analysis;
preferably, the crystal form B has a DSC pattern substantially as shown in Fig. 6;
preferably, the crystal form B has a weight loss of approximately 2.76% in a range of 21.34°C to 120°C, as shown by thermogravimetric analysis (TGA);
preferably, the crystal form B has a TGA pattern substantially as shown in Fig. 7.

**9.** The crystal form according to claim 6, wherein the crystal form is a crystal form C of the mono-L-tartrate of the compound of formula I, and the crystal form C has characteristic peaks at 17.88 ± 0.20°, 19.40 ± 0.20°, 21.38 ± 0.20° in an X-ray powder diffraction expressed at a 2θ angle using Cu-Kα radiation;

preferably, the crystal form C has characteristic peaks at 7.14 ± 0.20°, 17.88 ± 0.20°, 19.40 ± 0.20°, 20.06 ± 0.20°, 21.38 ± 0.20°, 23.76 ± 0.20°, 25.92 ± 0.20° in an X-ray powder diffraction expressed at a 2θ angle using Cu-Kα radiation;
preferably, the crystal form C has characteristic peaks at 3.58 ± 0.20°, 7.14 ± 0.20°, 13.96 ± 0.20°, 17.10 ± 0.20°, 17.88 ± 0.20°, 19.40 ± 0.20°, 20.06 ± 0.20°, 21.38 ± 0.20°, 25.92 ± 0.20°, 29.38 ± 0.20° in an X-ray powder diffraction expressed at a 2θ angle using Cu-Kα radiation;
preferably, the crystal form C has an X-ray powder diffraction expressed at a 2θ angle using Cu-Kα radiation as shown in Table 3, with an error range of ± 0.20°;
preferably, the crystal form C has an X-ray powder diffraction pattern substantially as shown in Fig. 8;
preferably, the crystal form C is a solvate of the *L*-tartrate of the compound of formula I, preferably an ethanol solvate of the *L*-tartrate of the compound of formula I;
preferably, the crystal form C has a first endothermic peak near a peak temperature of 129.45°C when heated and a second endothermic peak near a peak temperature of 151.90°C when heated, as shown by differential scanning calorimetry (DSC) analysis;
preferably, the crystal form C has a DSC pattern substantially as shown in Fig. 9;
preferably, the crystal form C has a weight loss of approximately 4.59% in a range of 21.47°C to 150°C, as shown by thermogravimetric analysis (TGA);
preferably, the crystal form C has a TGA pattern substantially as shown in Fig. 10.

**10.** The crystal form according to claim 6, wherein the crystal form is a crystal form D of the mono-*L*-tartrate of the compound of formula I, and the crystal form D has characteristic peaks at 3.50 ± 0.20°, 7.46 ± 0.20°, 23.04 ± 0.20° in an X-ray powder diffraction expressed at a 2θ angle using Cu-Kα radiation;

preferably, the crystal form D has characteristic peaks at 3.50 ± 0.20°, 6.92 ± 0.20°, 7.46 ± 0.20°, 17.22 ± 0.20°, 18.20 ± 0.20°, 19.88 ± 0.20°, 23.04 ± 0.20° in an X-ray powder diffraction expressed at a 2θ angle using Cu-Kα radiation;
preferably, the crystal form D has characteristic peaks at 3.50 ± 0.20°, 6.92 ± 0.20°, 7.46 ± 0.20°, 17.22 ± 0.20°, 18.20 ± 0.20°, 19.88 ± 0.20°, 20.76 ± 0.20°, 23.04 ± 0.20°, 25.62 ± 0.20° in an X-ray powder diffraction expressed at a 2θ angle using Cu-Kα radiation;
preferably, the crystal form D has an X-ray powder diffraction expressed at a 2θ angle using Cu-Kα radiation as shown in Table 4, with an error range of ± 0.20°;
preferably, the crystal form D has an X-ray powder diffraction pattern substantially as shown in Fig. 12;
preferably, the crystal form D is a solvate of the *L*-tartrate of the compound of formula I, preferably a tetrahydrofuran solvate of the *L*-tartrate of the compound of formula I;
preferably, the crystal form D has a first endothermic peak near a peak temperature of 94.15°C when heated, a second endothermic peak near a peak temperature of 118.79°C when heated, and a third endothermic peak near a peak temperature of 146.04°C when heated, as shown by differential scanning calorimetry (DSC) analysis;
preferably, the crystal form D has a DSC pattern substantially as shown in Fig. 13;
preferably, the crystal form D has a weight loss of approximately 5.25% in a range of 23.19°C to 120°C, as shown by thermogravimetric analysis (TGA);
preferably, the crystal form D has a TGA pattern substantially as shown in Fig. 14.

**11.** The crystal form according to claim 6, wherein the crystal form has unit cell parameters as follows:

monoclinic crystal form with space group $P2_1$,

$$a = 6.3430\ (5)\ \text{Å},$$

$$b = 8.8372\ (7)\ \text{Å},\ \beta = 96.577,$$

$$c = 24.809\ (2)\ \text{Å},$$

$$V = 1381.5\ (2)\ \text{Å}^3,$$

$$Z = 2.$$

**12.** A preparation method for the crystal form according to claim 7, wherein a preparation method I for the crystal form A of the mono-L-tartrate of the compound of formula I comprises stirring the mono-L-tartrate of the compound of formula I in a solvent to obtain the crystal form A;

preferably, the stirring is at a temperature of 20 to 80°C; the solvent is selected from an alcohol solvent, an ester solvent, a ketone solvent, an ether solvent, an alkane solvent, a halogenated hydrocarbon solvent, and a nitrile solvent, or a combination thereof;
preferably, the mass/volume ratio of the mono-*L*-tartrate of the compound of formula I to the solvent is 1 g:(20 to 40) mL;
a preparation method II for the crystal form A of the *L*-tartrate of the compound of formula I comprises dissolving the mono-L-tartrate of the compound of formula I in an alcohol solvent, followed by stirring with the addition of an antisolvent to obtain the crystal form A;
preferably, the alcohol solvent is selected from one of methanol, ethanol, propanol, and isopropanol; the anti solvent is one or more of an ether solvent or an ester solvent;
preferably, the mass/volume ratio of the mono-*L*-tartrate of the compound of formula I to the alcohol solvent and the antisolvent is 1 g:(10 to 30) mL:(80 to 120) mL;
more preferably, the preparation method I or II for the crystal form A further comprises post-processing steps such as filtration and drying.

**13.** A preparation method for the crystal form according to claim 8, wherein a preparation method I for the crystal form B of the mono-L-tartrate of the compound of formula I comprises placing the crystal form A under a high humidity condition to obtain the crystal form B; preferably, the high humidity condition is 80% to 100% RH, more preferably, 90% to 100% RH;

a preparation method II for the crystal form B of the mono-L-tartrate of the compound of formula I comprises dissolving the mono-L-tartrate of the compound of formula I in an alcohol solvent and evaporating the solvent to obtain the crystal form B;
preferably, the alcohol solvent is selected from one of methanol, ethanol, and isopropanol;
the dissolving is at a temperature of 15 to 45°C;
preferably, the mass/volume ratio of the mono-*L*-tartrate of the compound of formula I to the alcohol solvent is 1 g:(10 to 30) mL.

**14.** A preparation method for the crystal form according to claim 9, wherein the preparation method for the crystal form C of the mono-L-tartrate of the compound of formula I comprises dissolving the mono-L-tartrate of the compound of formula I in an alcohol solvent and crystallizing to obtain the crystal form C;

preferably, the alcohol solvent is selected from methanol, ethanol, and/or isopropanol;
preferably, the mass/volume ratio of the mono-*L*-tartrate of the compound of formula I to the alcohol solvent is 1 g:(20 to 40) mL;
more preferably, the preparation method for the crystal form C further comprises post-processing steps such as filtration and drying.

**15.** A preparation method for the crystal form according to claim 10, wherein the preparation method for the crystal form D of the mono-*L*-tartrate of the compound of formula I comprises dissolving the mono-*L*-tartrate of the compound of formula I in an ether solvent, heating with stirring, and cooling to precipitate a solid to obtain the crystal form D;

preferably, the ether solvent is selected from one of diethyl ether, tetrahydrofuran, and methyl *tert*-butyl ether; the heating is at a temperature of 30°C to 80°C; the cooling is at a temperature of 20°C to 30°C; the mass/volume ratio of the mono-*L*-tartrate of the compound of formula I to the ether solvent is 1 g:(20 to 40) mL;
more preferably, the preparation method for the crystal form D comprises post-processing steps such as filtration and drying.

**16.** A preparation method for the crystal form according to claim 11, comprising dissolving the mono-*L*-tartrate of the compound of formula I in a solvent D and then diffusing in an atmosphere of a solvent E;

the solvent D is selected from an alcohol solvent, such as methanol, ethanol, *n*-propanol, isopropanol, *n*-butanol, neopentyl alcohol, or a combination thereof;
the solvent E is selected from an ester solvent, an ether solvent, an alkane solvent, or a combination thereof; the ester solvent may be selected from an organic carboxylic acid ester, such as ethyl acetate and isopropyl acetate; the ether may be selected from an ether having 2 to 6 carbon atoms, such as diethyl ether, propyl ether, isopropyl ether, *tert*-butyl ether, and methyl tert-butyl ether; the alkane may be selected from a hydrocarbon having 1 to 8 carbon atoms, such as n-hexane and n-heptane.

**17.** A pharmaceutical composition, comprising the pharmaceutically acceptable salt of the compound of formula I according to any one of claims 1 to 4 or the crystal form according to any one of claims 6 to 11, and optionally a pharmaceutically acceptable pharmaceutical excipient; preferably, the pharmaceutical composition is in the form of a preparation.

**18.** A use of the pharmaceutically acceptable salt of the compound of formula I according to any one of claims 1 to 4, the crystal form according to any one of claims 6 to 11, or the pharmaceutical composition according to claim 17 in the manufacture of a drug for preventing and/or treating a disease or condition related to a Vanin enzyme inhibitor; preferably, the disease or condition related to the Vanin enzyme inhibitor comprises one or more of autoimmune disease, inflammatory disease, allergic disease, metabolic disease, infection-based disease, fibrotic disease, cardiovascular disease, respiratory disease, renal disease, dermatological disease, hepatic disease, gastrointestinal disease, oral disease, and hematopoietic disease; for another example, Crohn's disease, inflammatory bowel disease, and ulcerative colitis.

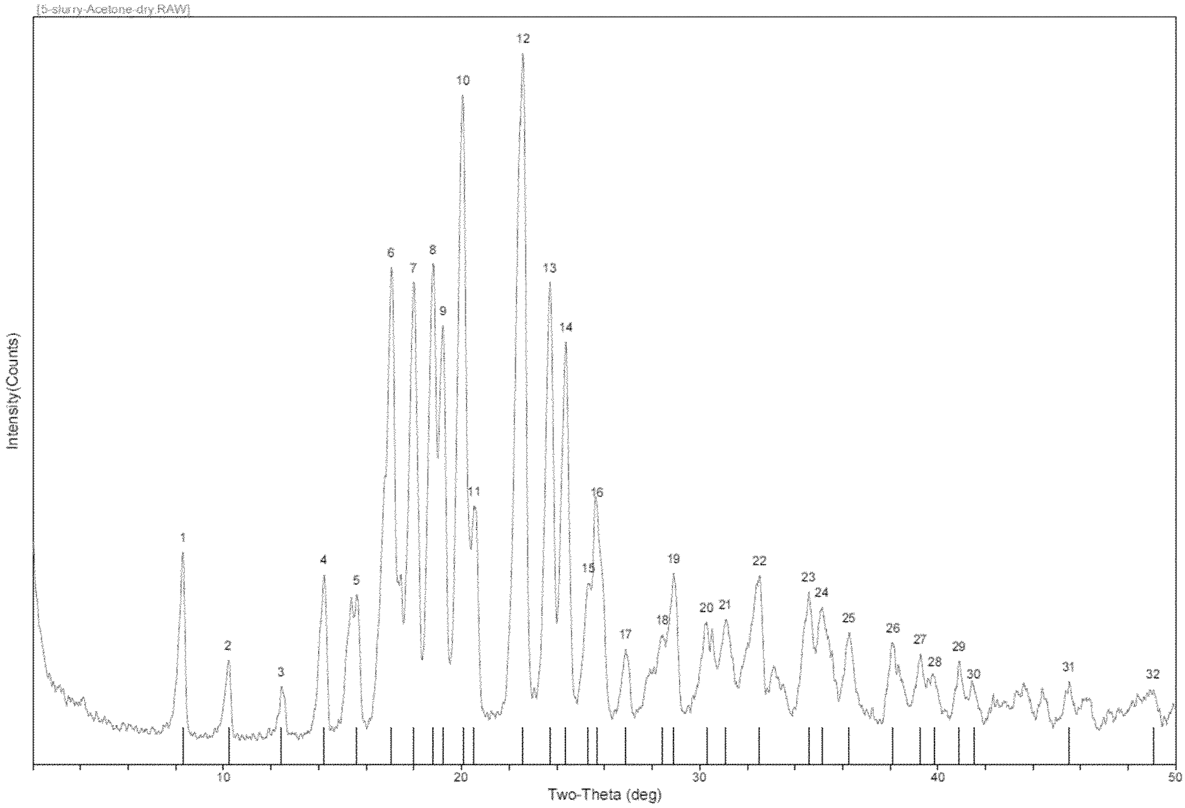
[5-slurry-Acetone-dry.RAW]
Intensity(Counts)
Two-Theta (deg)
10
20
30
40
50


Fig. 1

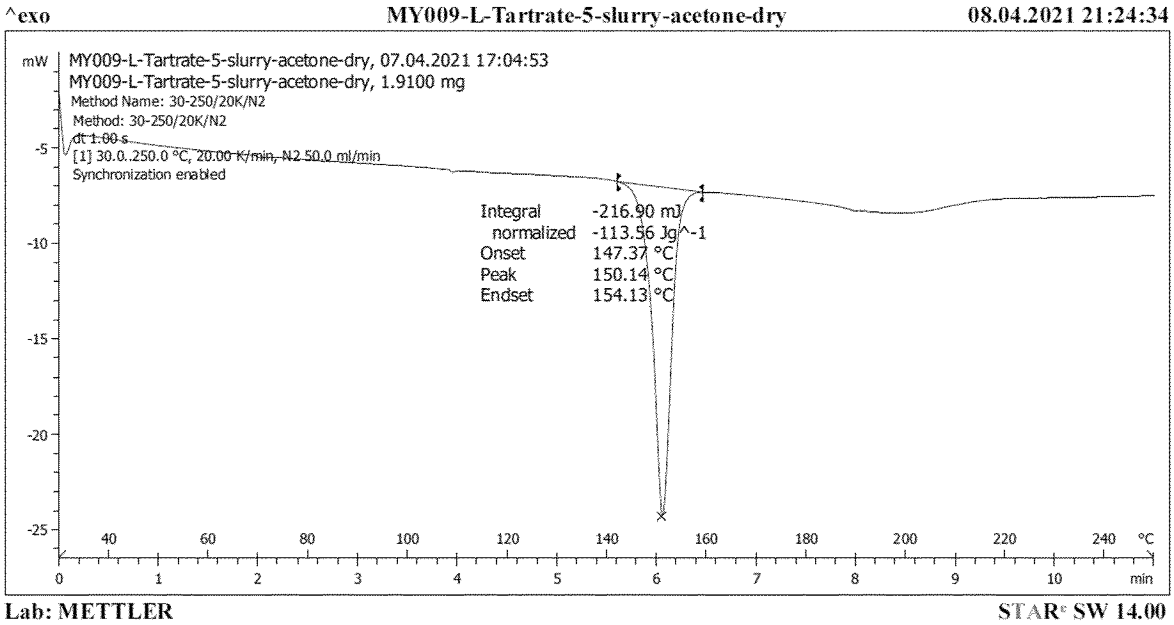
^exo
MY009-L-Tartrate-5-slurry-acetone-dry
08.04.2021 21:24:34
mW
MY009-L-Tartrate-5-slurry-acetone-dry, 07.04.2021 17:04:53
MY009-L-Tartrate-5-slurry-acetone-dry, 1.9100 mg
Method Name: 30-250/20K/N2
Method: 30-250/20K/N2
dt 1.00 s
[1] 30.0..250.0 °C, 20.00 K/min, N2 50.0 ml/min
Synchronization enabled
Integral -216.90 mJ
normalized -113.56 Jg^-1
Onset 147.37 °C
Peak 150.14 °C
Endset 154.13 °C
Lab: METTLER
STARe SW 14.00


Fig. 2

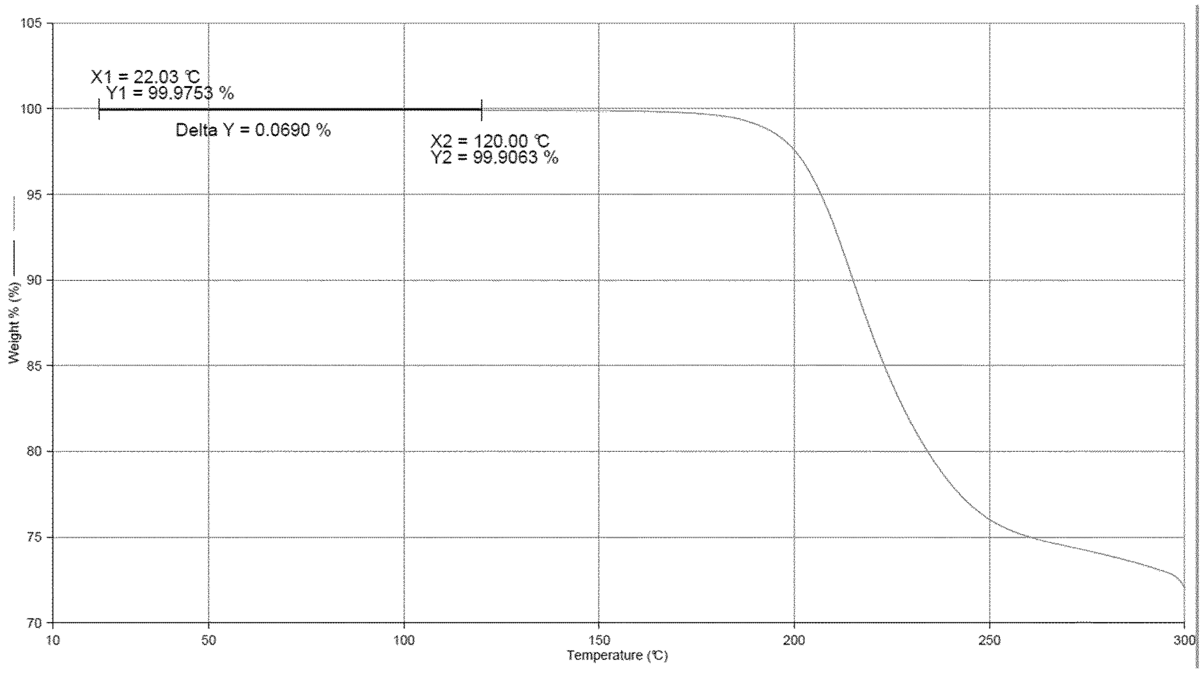
105
100
95
90
85
80
75
70
Weight % (%)
10
50
100
150
200
250
300
Temperature (℃)
X1 = 22.03 ℃
Y1 = 99.9753 %
Delta Y = 0.0690 %
X2 = 120.00 ℃
Y2 = 99.9063 %

Fig. 3

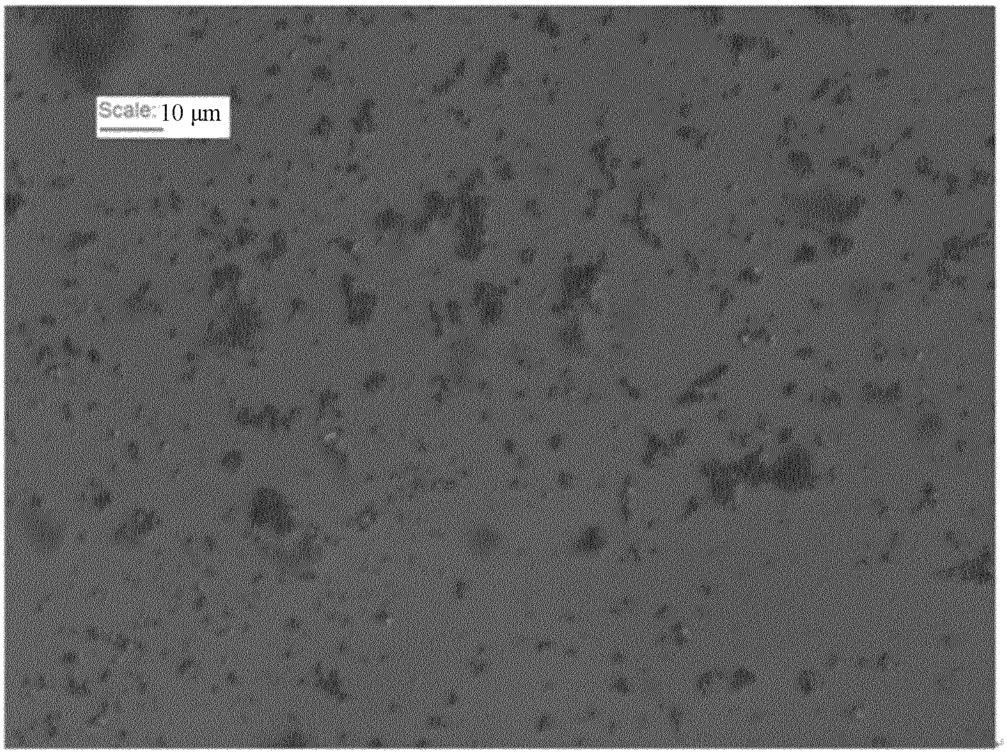
Scale: 10 μm

Fig. 4

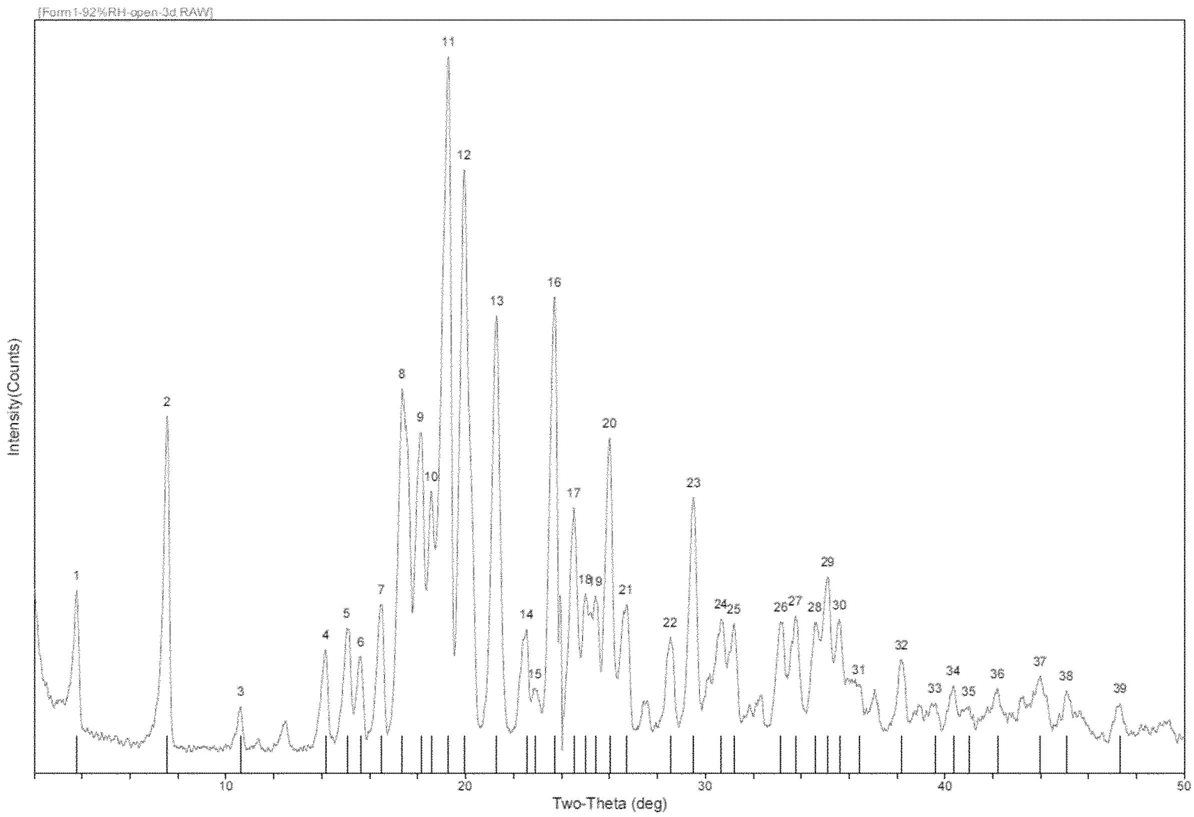

[Form1-92%RH-open-3d.RAW]
Intensity(Counts)
Two-Theta (deg)
10
20
30
40
50


Fig. 5

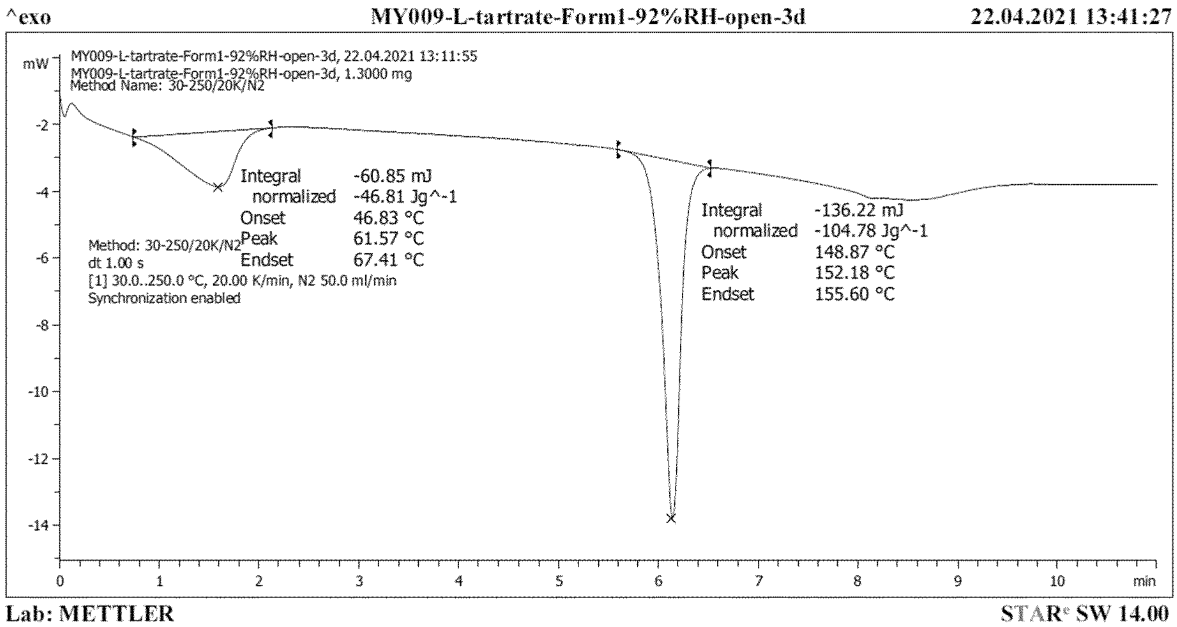

^exo
MY009-L-tartrate-Form1-92%RH-open-3d
22.04.2021 13:41:27
mW
MY009-L-tartrate-Form1-92%RH-open-3d, 22.04.2021 13:11:55
MY009-L-tartrate-Form1-92%RH-open-3d, 1.3000 mg
Method Name: 30-250/20K/N2
Integral -60.85 mJ
normalized -46.81 Jg^-1
Onset 46.83 °C
Peak 61.57 °C
Endset 67.41 °C
Method: 30-250/20K/N2
dt 1.00 s
[1] 30.0..250.0 °C, 20.00 K/min, N2 50.0 ml/min
Synchronization enabled
Integral -136.22 mJ
normalized -104.78 Jg^-1
Onset 148.87 °C
Peak 152.18 °C
Endset 155.60 °C
min
Lab: METTLER
STARe SW 14.00


Fig. 6

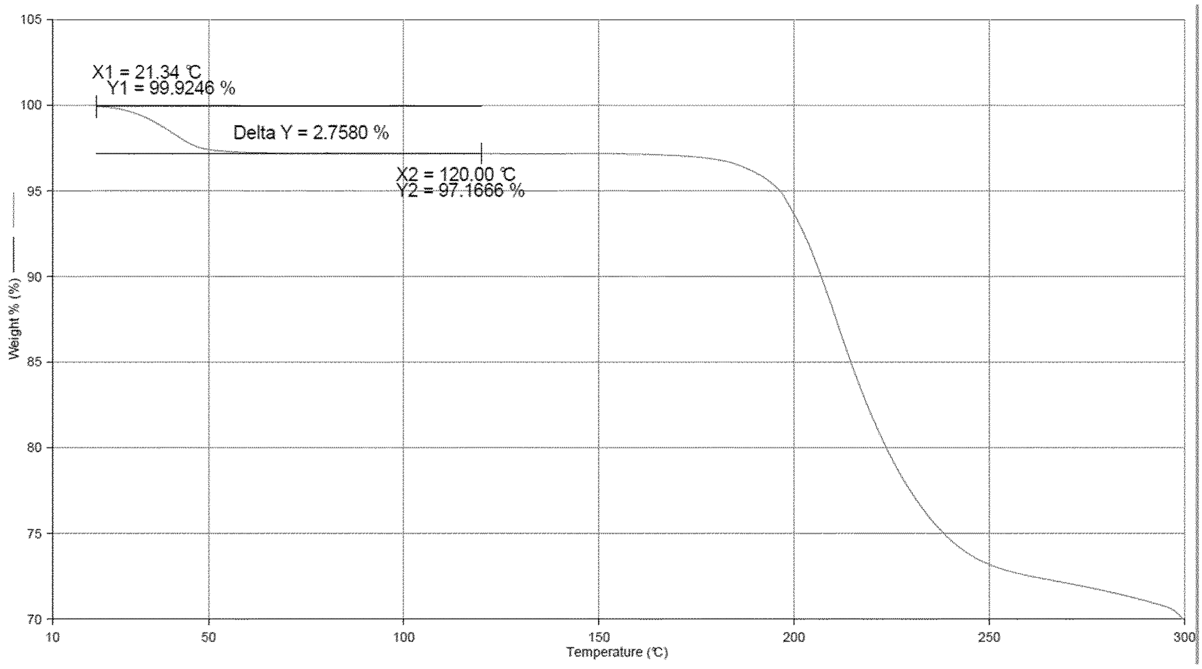
X1 = 21.34 ℃
Y1 = 99.9246 %
Delta Y = 2.7580 %
X2 = 120.00 ℃
Y2 = 97.1666 %
Weight % (%)
Temperature (℃)
105
100
95
90
85
80
75
70
10
50
100
150
200
250
300

Fig. 7

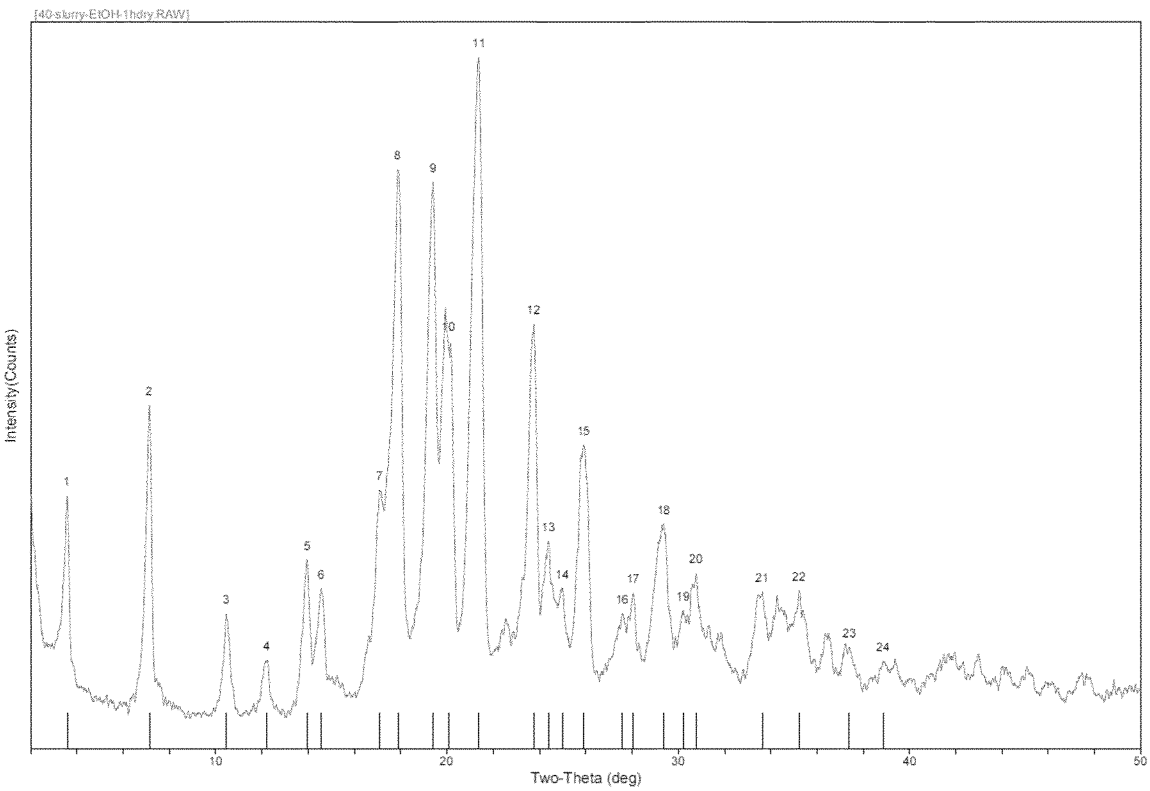
[40-slurry-EtOH-1hdry.RAW]
Intensity(Counts)
Two-Theta (deg)
10
20
30
40
50

Fig. 8

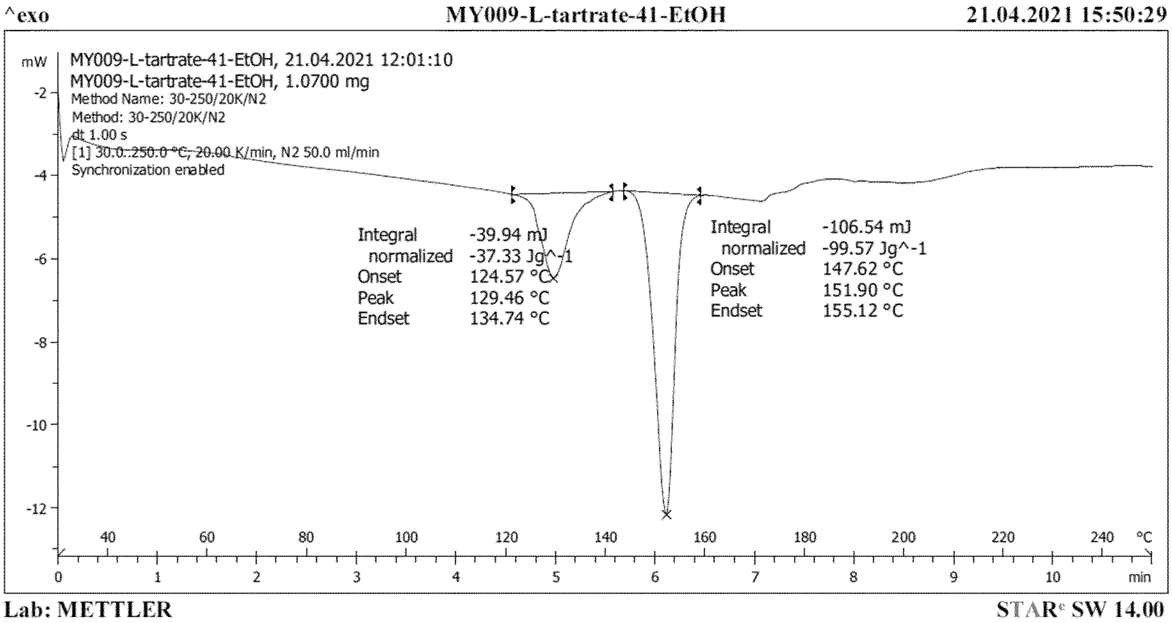
^exo
MY009-L-tartrate-41-EtOH
21.04.2021 15:50:29
MY009-L-tartrate-41-EtOH, 21.04.2021 12:01:10
MY009-L-tartrate-41-EtOH, 1.0700 mg
Method Name: 30-250/20K/N2
Method: 30-250/20K/N2
dt 1.00 s
[1] 30.0..250.0 °C, 20.00 K/min, N2 50.0 ml/min
Synchronization enabled
Integral -39.94 mJ
normalized -37.33 Jg^-1
Onset 124.57 °C
Peak 129.46 °C
Endset 134.74 °C
Integral -106.54 mJ
normalized -99.57 Jg^-1
Onset 147.62 °C
Peak 151.90 °C
Endset 155.12 °C
mW
°C
min
Lab: METTLER
STARe SW 14.00

Fig. 9

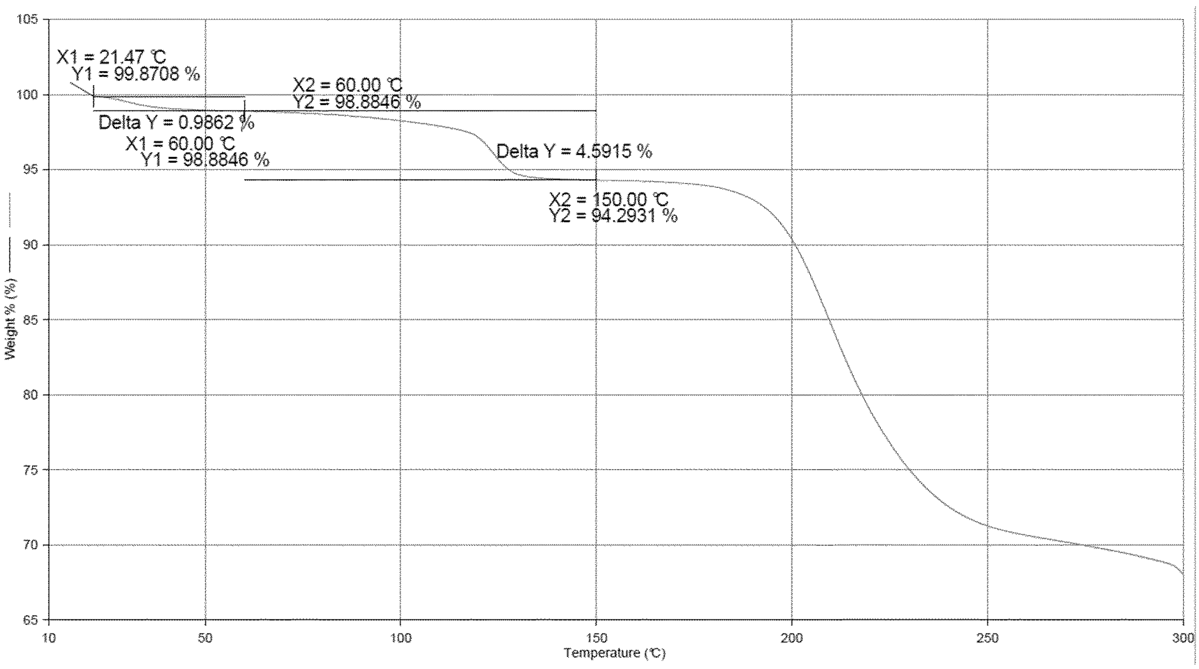
X1 = 21.47 ℃
Y1 = 99.8708 %
X2 = 60.00 ℃
Y2 = 98.8846 %
Delta Y = 0.9862 %
X1 = 60.00 ℃
Y1 = 98.8846 %
Delta Y = 4.5915 %
X2 = 150.00 ℃
Y2 = 94.2931 %
Weight % (%)
Temperature (℃)

Fig. 10

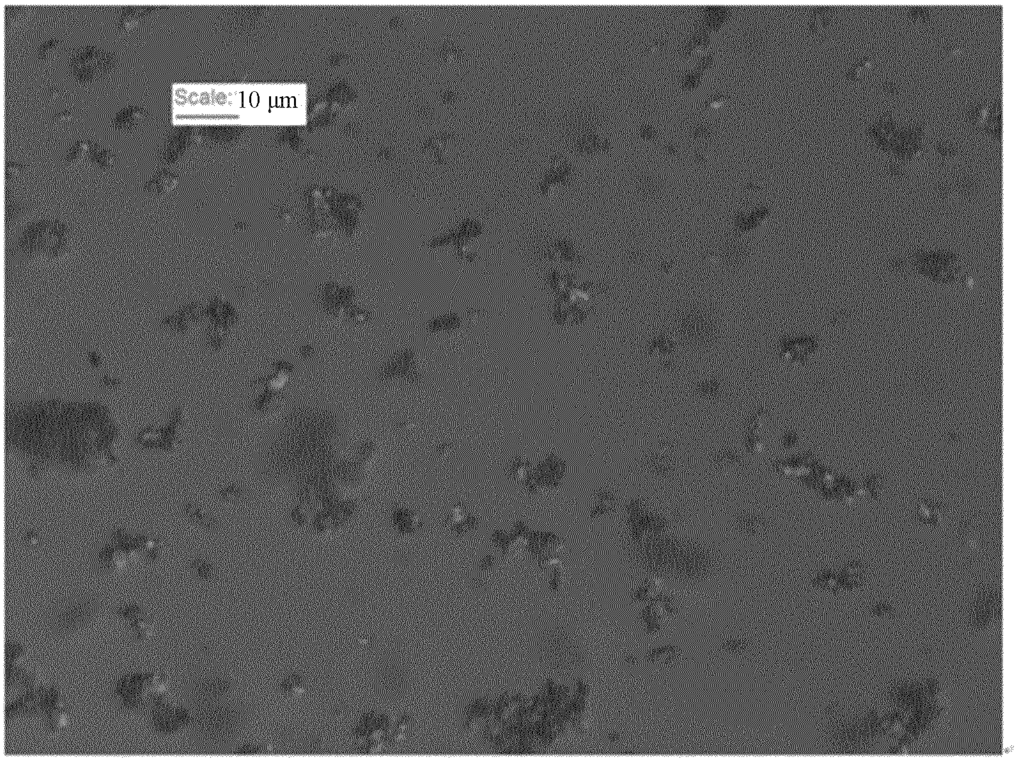
Scale: 10 μm

Fig. 11

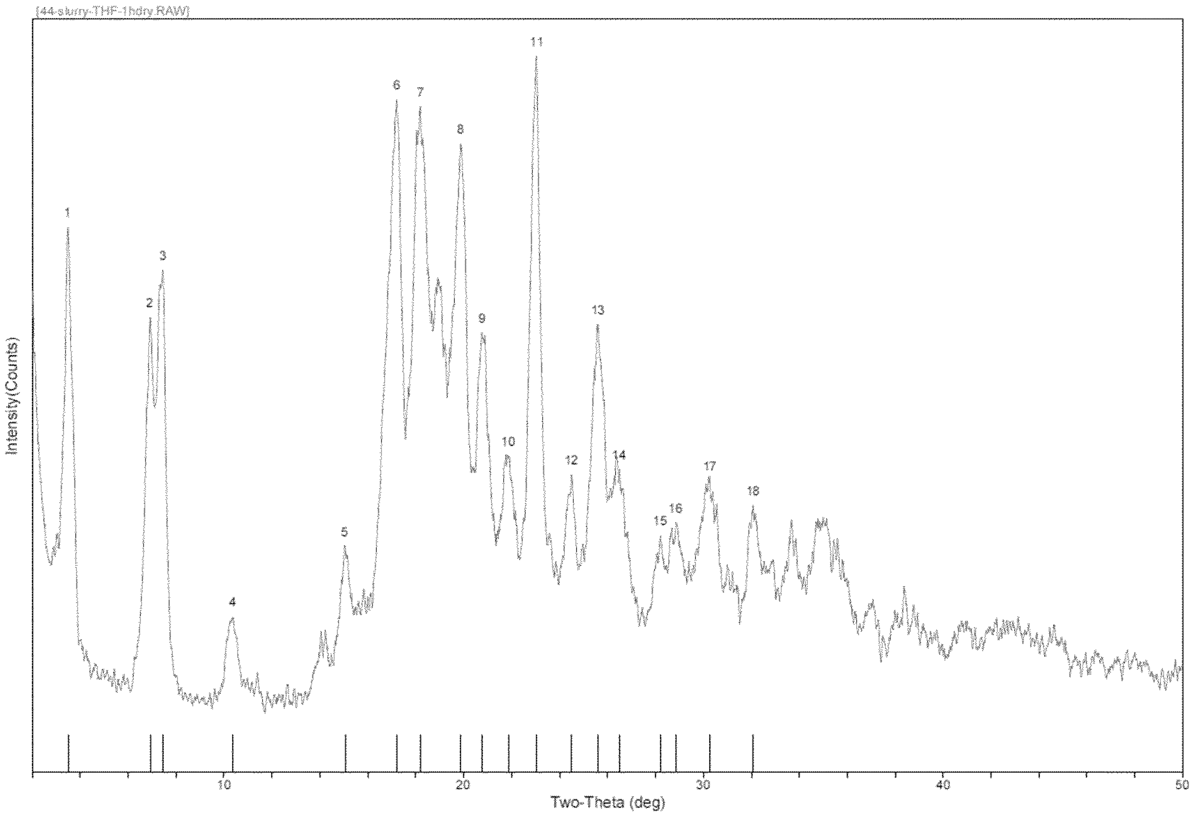
[44-slurry-THF-1hdry.RAW]
Intensity(Counts)
Two-Theta (deg)
1
2
3
4
5
6
7
8
9
10
11
12
13
14
15
16
17
18
10
20
30
40
50

Fig. 12

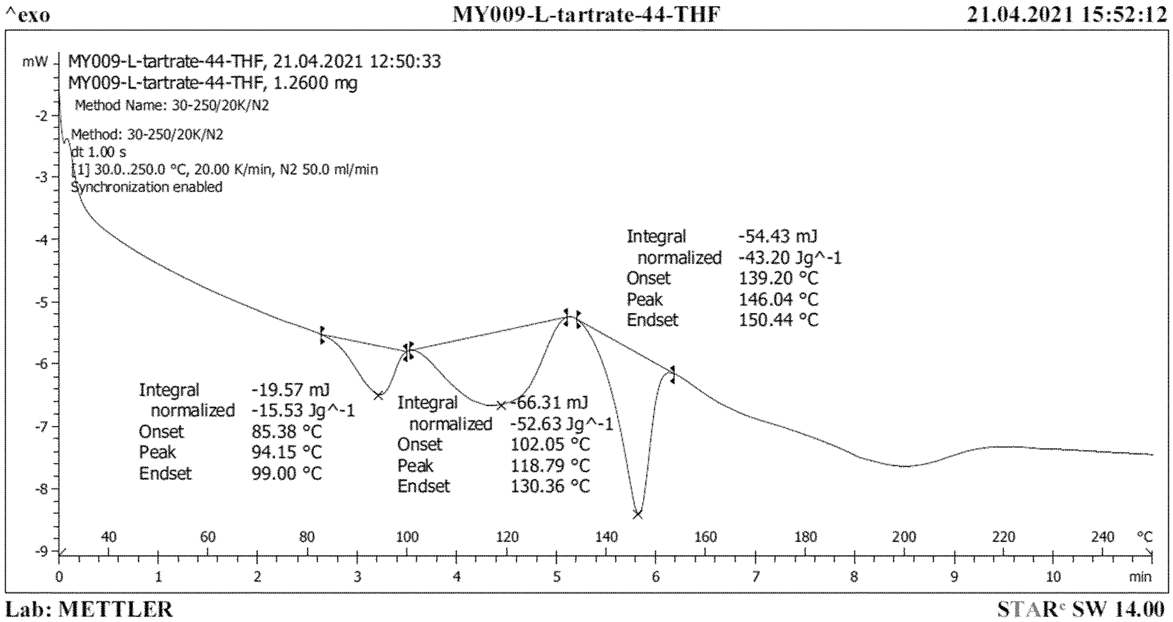
^exo
MY009-L-tartrate-44-THF
21.04.2021 15:52:12
MY009-L-tartrate-44-THF, 21.04.2021 12:50:33
MY009-L-tartrate-44-THF, 1.2600 mg
Method Name: 30-250/20K/N2
Method: 30-250/20K/N2
dt 1.00 s
[1] 30.0..250.0 °C, 20.00 K/min, N2 50.0 ml/min
Synchronization enabled
Integral -54.43 mJ
normalized -43.20 Jg^-1
Onset 139.20 °C
Peak 146.04 °C
Endset 150.44 °C
Integral -19.57 mJ
normalized -15.53 Jg^-1
Onset 85.38 °C
Peak 94.15 °C
Endset 99.00 °C
Integral -66.31 mJ
normalized -52.63 Jg^-1
Onset 102.05 °C
Peak 118.79 °C
Endset 130.36 °C
mW
°C
min
Lab: METTLER
STARe SW 14.00

Fig. 13

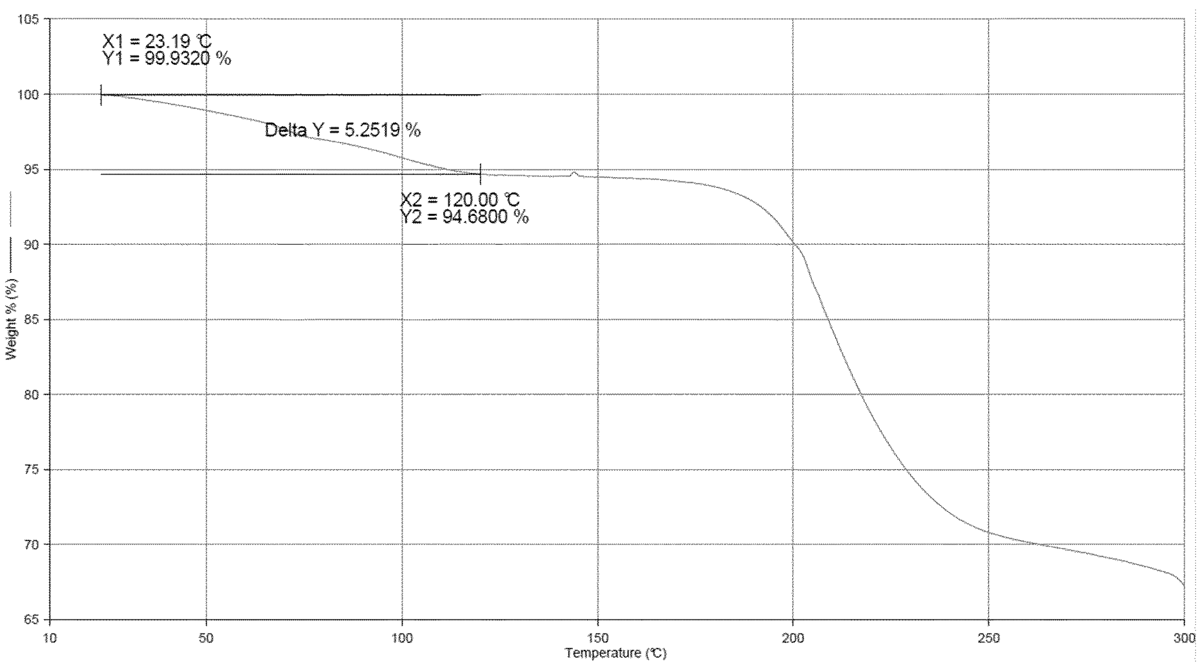
X1 = 23.19 °C
Y1 = 99.9320 %
Delta Y = 5.2519 %
X2 = 120.00 °C
Y2 = 94.6800 %
Weight % (%)
Temperature (°C)

Fig. 14

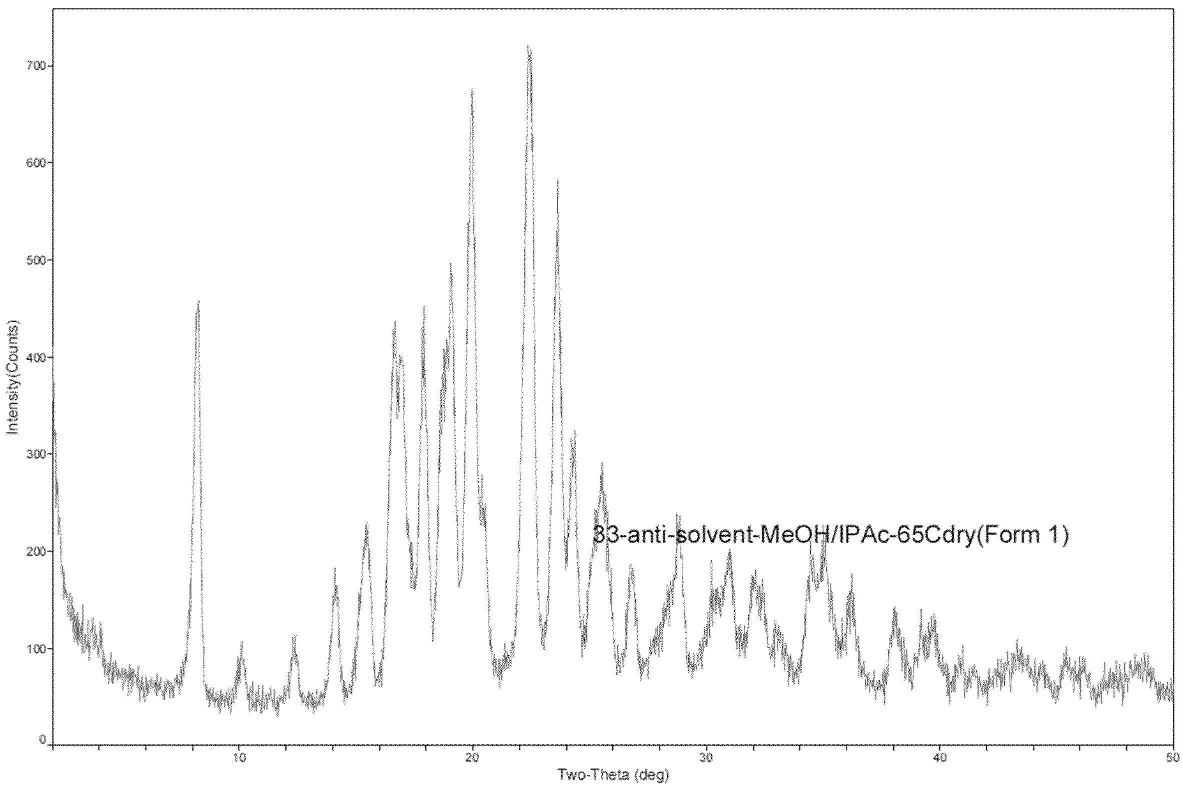
Intensity(Counts)
Two-Theta (deg)
33-anti-solvent-MeOH/IPAc-65Cdry(Form 1)

Fig. 15

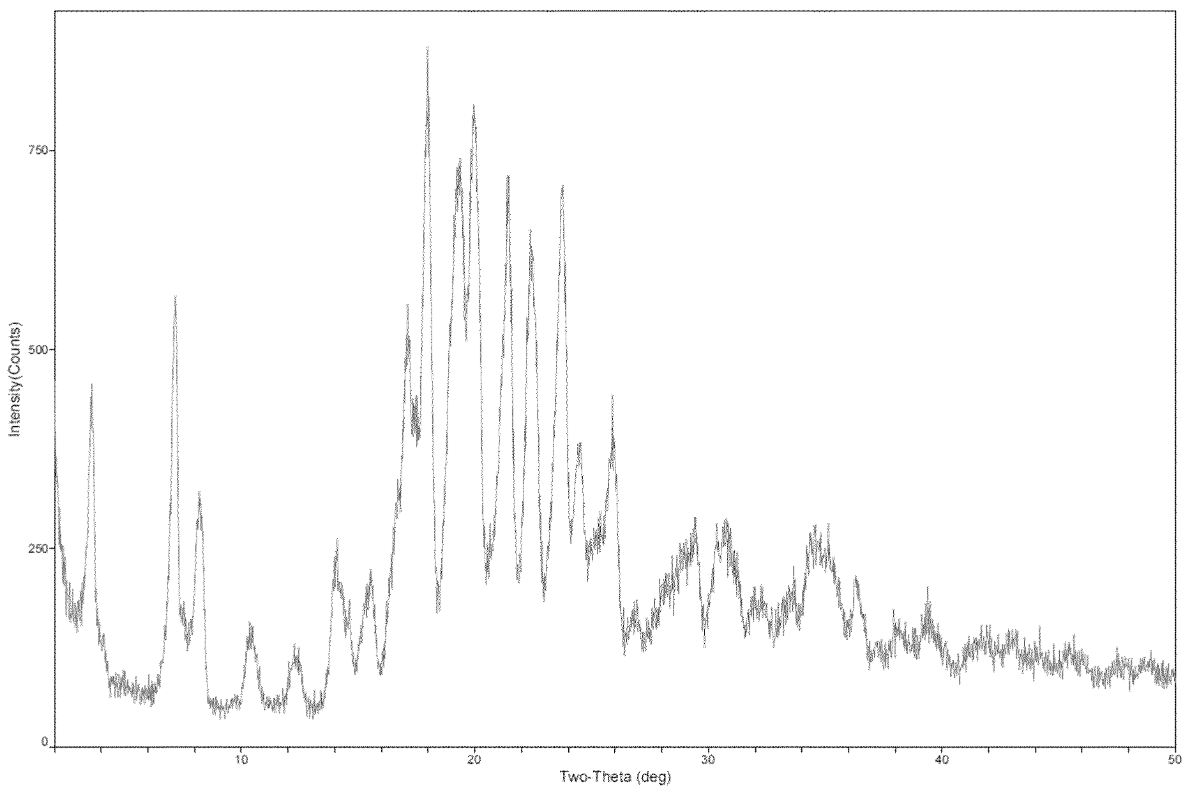
Intensity(Counts)
Two-Theta (deg)

Fig. 16

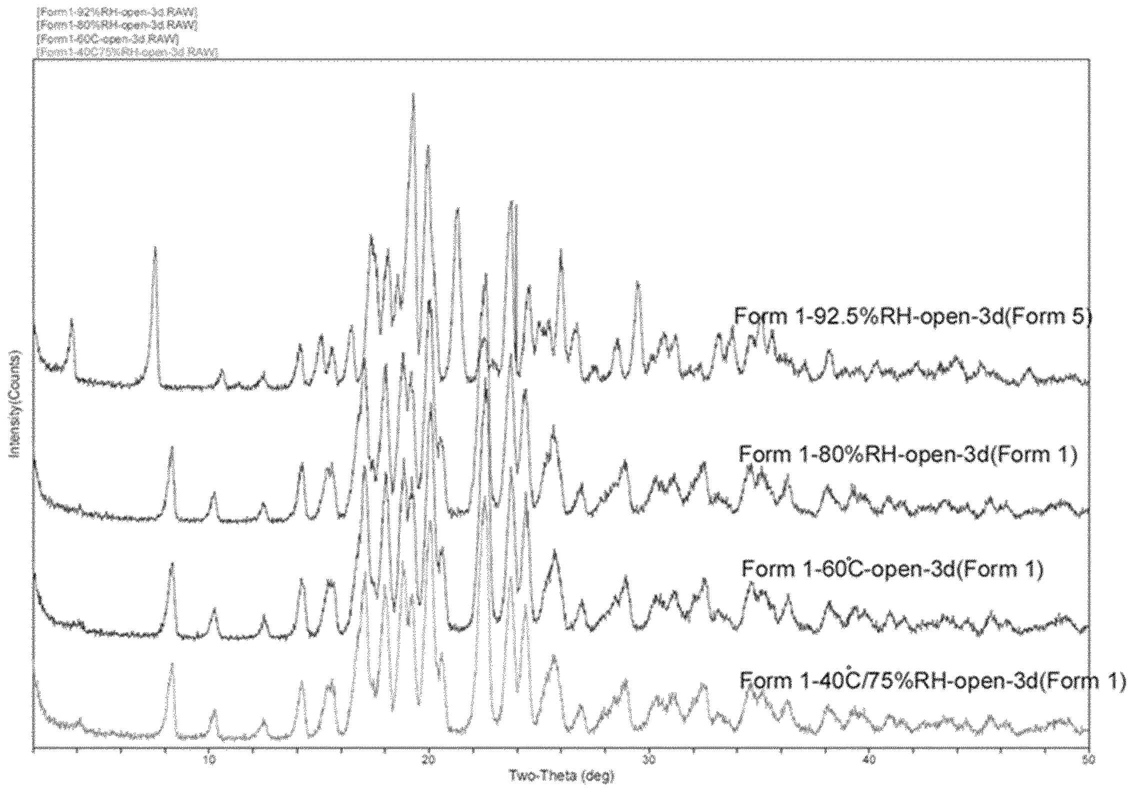
[Form1-92%RH-open-3d.RAW]
[Form1-80%RH-open-3d.RAW]
[Form1-60C-open-3d.RAW]
[Form1-40C75%RH-open-3d.RAW]
Intensity(Counts)
Form 1-92.5%RH-open-3d(Form 5)
Form 1-80%RH-open-3d(Form 1)
Form 1-60°C-open-3d(Form 1)
Form 1-40°C/75%RH-open-3d(Form 1)
10
20
30
40
50
Two-Theta (deg)

Fig. 17

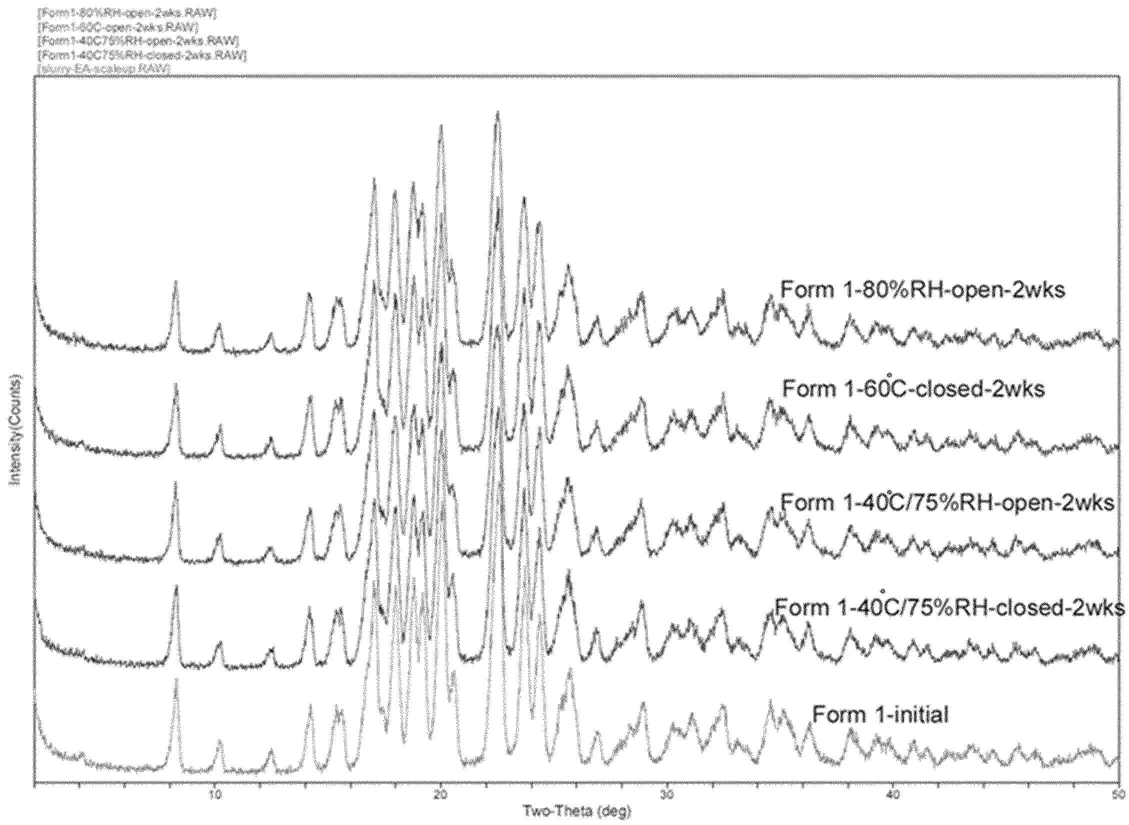
[Form1-80%RH-open-2wks.RAW]
[Form1-60C-open-2wks.RAW]
[Form1-40C75%RH-open-2wks.RAW]
[Form1-40C75%RH-closed-2wks.RAW]
[slurry-EA-scaleup.RAW]
Intensity(Counts)
Form 1-80%RH-open-2wks
Form 1-60°C-closed-2wks
Form 1-40°C/75%RH-open-2wks
Form 1-40°C/75%RH-closed-2wks
Form 1-initial
10
20
30
40
50
Two-Theta (deg)

Fig. 18

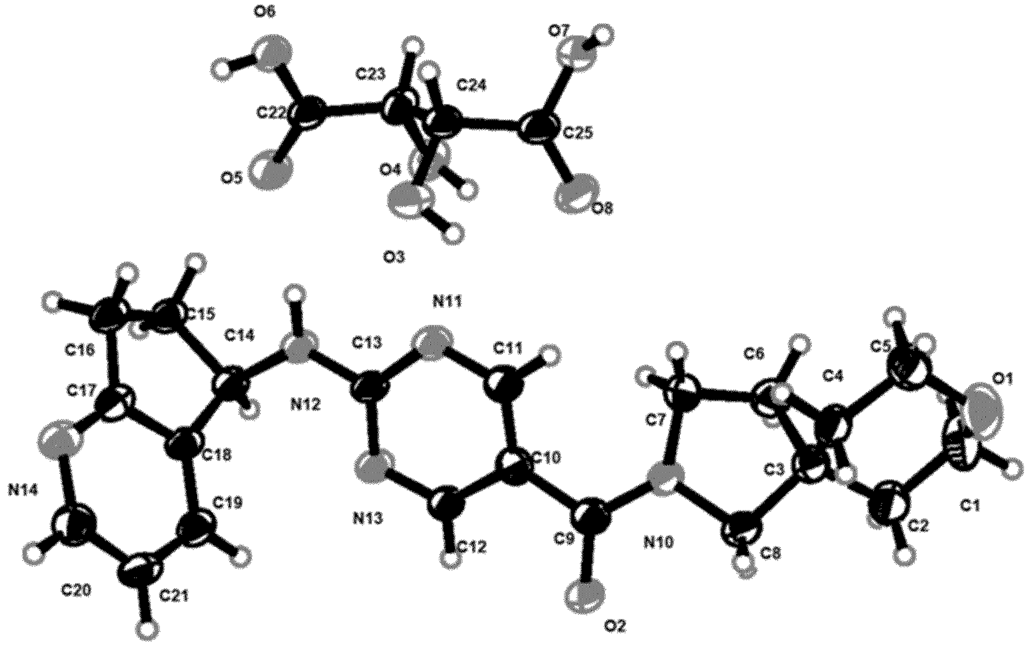

Fig. 19

**INTERNATIONAL SEARCH REPORT**

International application No.
**PCT/CN2023/082191**

**A. CLASSIFICATION OF SUBJECT MATTER**

C07D 491/107(2006.01)i; C07C 59/255(2006.01)i; C07C 51/41(2006.01)i; A61K 31/444(2006.01)i; A61P 37/02(2006.01)i; A61P 37/08(2006.01)i; A61P 29/00(2006.01)i; A61P 31/00(2006.01)i; A61P 3/00(2006.01)i; A61P 9/00(2006.01)i; A61P 11/00(2006.01)i; A61P 13/12(2006.01)i; A61P 17/00(2006.01)i

According to International Patent Classification (IPC) or to both national classification and IPC

**B. FIELDS SEARCHED**

Minimum documentation searched (classification system followed by classification symbols)

IPC: C07D,C07C,A61K,A61P

Documentation searched other than minimum documentation to the extent that such documents are included in the fields searched

Electronic data base consulted during the international search (name of data base and, where practicable, search terms used)

CNABS, CNTXT, VEN, ENTXT, ENTXTC, CNKI, 万方, WANFANG, 百度, BAIDU, caplus, marpat, registry: 美悦, 姚元山, 田勇, 栾林波, 陈永凯, 王朝东, 血管非炎性分子, Vanin 3d 酶, Vanin 3d enzyme, 心血管, 盐, structural formula search.

**C. DOCUMENTS CONSIDERED TO BE RELEVANT**

| Category* | Citation of document, with indication, where appropriate, of the relevant passages | Relevant to claim No. |
|---|---|---|
| PX | WO 2022063197 A1 (SHANGHAI MEIYUE BIOTECH DEVELOPMENT CO., LTD.) 31 March 2022 (2022-03-31)<br>description, embodiment 2, and description, pp. 2 and 12-13 | 1-18 |
| X | CN 113226462 A (BOEHRINGER INGELHEIM INTERNATIONAL GMBH) 06 August 2021 (2021-08-06)<br>description, paragraphs [0014]-[0197] and [0225]-[0228] | 1-18 |
| A | CN 112601748 A (BOEHRINGER INGELHEIM INTERNATIONAL GMBH) 02 April 2021 (2021-04-02)<br>entire document | 1-18 |
| A | CN 113166104 A (BOEHRINGER INGELHEIM INTERNATIONAL GMBH) 23 July 2021 (2021-07-23)<br>entire document | 1-18 |

☐ Further documents are listed in the continuation of Box C.   ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **21 June 2023** | **28 June 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2023/082191**

| Patent document cited in search report | Publication date (day/month/year) | Patent family member(s) | Publication date (day/month/year) |
|---|---|---|---|
| WO 2022063197 A1 | 31 March 2022 | WO 2022063333 A1 | 31 March 2022 |
| | | CA 3193204 A1 | 31 March 2022 |
| | | KR 20230049673 A | 13 April 2023 |
| | | TW 202216722 A | 01 May 2022 |
| CN 113226462 A | 06 August 2021 | EP 3890828 A1 | 13 October 2021 |
| | | US 2022048889 A1 | 17 February 2022 |
| | | KR 20210099093 A | 11 August 2021 |
| | | BR 112021010823 A2 | 24 August 2021 |
| | | WO 2020114947 A1 | 11 June 2020 |
| | | CA 3120037 A1 | 11 June 2020 |
| | | JP 2022510351 A | 26 January 2022 |
| | | JP 7195436 B2 | 23 December 2022 |
| | | AU 2019392524 A1 | 15 July 2021 |
| CN 112601748 A | 02 April 2021 | AU 2019326933 A1 | 28 January 2021 |
| | | IL 280898 A | 29 April 2021 |
| | | CR 20210101 A | 30 April 2021 |
| | | SG 11202101749 VA | 30 March 2021 |
| | | AR 116022 A1 | 25 March 2021 |
| | | JOP 20210034 A1 | 25 February 2021 |
| | | CL 2021000288 A1 | 20 August 2021 |
| | | KR 20210052500 A | 10 May 2021 |
| | | MA 53479 A | 04 May 2022 |
| | | MX 2021002323 A | 28 April 2021 |
| | | ES 2932557 T3 | 20 January 2023 |
| | | EP 3844161 A1 | 07 July 2021 |
| | | EP 3844161 B1 | 09 November 2022 |
| | | PE 20210478 A1 | 11 March 2021 |
| | | CO 2021002225 A2 | 08 March 2021 |
| | | WO 2020043658 A1 | 05 March 2020 |
| | | DOP 2021000021 A | 28 February 2021 |
| | | US 2020069663 A1 | 05 March 2020 |
| | | US 10864201 B2 | 15 December 2020 |
| | | PL 3844161 T3 | 13 March 2023 |
| | | US 2021059992 A1 | 04 March 2021 |
| | | CA 3106513 A1 | 05 March 2020 |
| | | DK 3844161 T3 | 12 December 2022 |
| | | JP 2021535154 A | 16 December 2021 |
| | | JP 7148709 B2 | 05 October 2022 |
| | | PH 12021550269 A1 | 11 October 2021 |
| | | EA 202190588 A1 | 23 June 2021 |
| | | TW 202023550 A | 01 July 2020 |
| | | BR 112021000650 A2 | 13 April 2021 |
| CN 113166104 A | 23 July 2021 | US 2022048885 A1 | 17 February 2022 |
| | | SG 11202104437 XA | 28 May 2021 |
| | | WO 2020114943 A1 | 11 June 2020 |
| | | US 2020172508 A1 | 04 June 2020 |
| | | US 11078182 B2 | 03 August 2021 |
| | | CO 2021007173 A2 | 21 June 2021 |
| | | CL 2021003568 A1 | 04 November 2022 |
| | | AR 117229 A1 | 21 July 2021 |

Form PCT/ISA/210 (patent family annex) (July 2022)

**INTERNATIONAL SEARCH REPORT**
**Information on patent family members**

International application No.
**PCT/CN2023/082191**

| Patent document cited in search report | Publication date (day/month/year) | Patent family member(s) | | | Publication date (day/month/year) |
|---|---|---|---|---|---|
| | | IL | 283397 | A | 29 July 2021 |
| | | PH | 12021551280 | A1 | 06 December 2021 |
| | | MA | 54378 | A | 09 March 2022 |
| | | JOP | 20210128 | A1 | 30 January 2023 |
| | | EP | 3891139 | A1 | 13 October 2021 |
| | | BR | 112021007915 | A2 | 27 July 2021 |
| | | ECSP | 21040278 | A | 30 July 2021 |
| | | PE | 20211769 | A1 | 07 September 2021 |
| | | DOP | 2021000071 | A | 31 May 2021 |
| | | JOP | 20210127 | A1 | 30 January 2023 |
| | | JP | 2022188014 | A | 20 December 2022 |
| | | EA | 202191476 | A1 | 26 October 2021 |
| | | CL | 2021001198 | A1 | 24 December 2021 |
| | | MX | 2021006520 | A | 07 July 2021 |
| | | TW | 202039461 | A | 01 November 2020 |
| | | KR | 20210100150 | A | 13 August 2021 |
| | | CA | 3116830 | A1 | 11 June 2020 |
| | | JP | 2022509317 | A | 20 January 2022 |
| | | JP | 7130873 | B2 | 05 September 2022 |
| | | CR | 20210294 | A | 13 August 2021 |
| | | AU | 2019391279 | A1 | 13 May 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202210272562
- CN 202211104893
- CN 2021110954656
- WO 2022063197 A1

### Non-patent literature cited in the description


- **BERRUYER C et al.** Vanin-1-/- mice exhibit a glutathione mediated tissue resistance to oxidative stress. *Mol. Cell Biol.*, 2004, vol. 24, 7214-7224
- **BERRUYER C et al.** Vanin-1 licenses inflammatory mediator production by gut epithelial cells and controls colitis by antagonizing peroxisome proliferator-activated receptor $\gamma$ activity.. *J. Exp. Med.*, 2006, vol. 203, 2817-2827
- **ROMMELAERE S et al.** PPARalpha regulates the production of serum Vanin-1 by liver.. *FEBS Lett.*, 15 November 2013, vol. 587 (22), 3742-8